(19) Europäisches Patentamt
European Patent Office
Office européen des brevets

(11) EP 1 633 882 B1

(12) **EUROPEAN PATENT SPECIFICATION**

(45) Date of publication and mention of the grant of the patent:
**14.05.2008 Bulletin 2008/20**

(21) Application number: **04733251.5**

(22) Date of filing: **15.05.2004**

(51) Int Cl.:
***C12Q 1/37** (2006.01)* ***C12Q 1/68** (2006.01)*
***G01N 33/68** (2006.01)* ***G01N 33/573** (2006.01)*
***A61K 38/00** (2006.01)* ***A61K 39/00** (2006.01)*

(86) International application number:
**PCT/EP2004/005252**

(87) International publication number:
**WO 2004/106540 (09.12.2004 Gazette 2004/50)**

(54) **DIAGNOSTICS AND THERAPEUTICS FOR DISEASES ASSOCIATED WITH PUROMYCIN-INSENSITIVE LEUCYL-SPECIFIC AMINOPEPTIDASE (PILS)**

DIAGNOSTIK- UND THERAPIE- VERFAHREN VON MIT PILS VERBUNDENEN KRANKHEITEN

DIAGNOSTIC ET THERAPEUTIQUE DE MALADIES ASSOCIEES A UNE AMINOPEPTIDASE SPECIFIQUE A LEUCYLE INSENSIBLE A LA PUROMYCINE (PILS)

(84) Designated Contracting States:
**AT BE BG CH CY CZ DE DK EE ES FI FR GB GR HU IE IT LI LU MC NL PL PT RO SE SI SK TR**

(30) Priority: **28.05.2003 EP 03011962**

(43) Date of publication of application:
**15.03.2006 Bulletin 2006/11**

(73) Proprietor: **Bayer HealthCare AG**
**51368 Leverkusen (DE)**

(72) Inventors:
- **GOLZ, Stefan**
  **45326 Essen (DE)**
- **BRÜGGEMEIER, Ulf**
  **42799 Leichlingen (DE)**
- **GEERTS, Andreas**
  **42113 Wuppertal (DE)**

(56) References cited:
**WO-A-00/73454** **WO-A-01/22920**
**WO-A-01/64856** **WO-A-01/90304**

- **YAMAMOTO NAO ET AL: "Identification of 33 polymorphisms in the adipocyte-derived leucine aminopeptidase (ALAP) gene and possible association with hypertension" HUMAN MUTATION, vol. 19, no. 3, 2002, pages 251-257, XP008034131 ISSN: 1059-7794**
- **HATTORI AKIRA ET AL: "Characterization of recombinant human adipocyte-derived leucine aminopeptidase expressed in Chinese hamster ovary cells" JOURNAL OF BIOCHEMISTRY (TOKYO), vol. 128, no. 5, November 2000 (2000-11), pages 755-762, XP001194748 ISSN: 0021-924X cited in the application**
- **HALLBERG PAR ET AL: "Adipocyte-derived leucine aminopeptidase genotype and response to antihypertensive therapy." BMC CARDIOVASCULAR DISORDERS [ELECTRONIC RESOURCE]. 18 SEP 2003, vol. 3, no. 1, 18 September 2003 (2003-09-18), page 11, XP001199495 ISSN: 1471-2261**
- **LIEBMAN, M.N.: DRUG DISCOVERY TODAY, vol. 7, no. 20(Suppl.), 2002, pages S197-S203,**
- **FEHNIGER T.E. ET AL.: DRUG DISCOVERY TODAY: TECHNOLOGIES, vol. 2, no. 4, 2005, pages 345-351,**
- **SIEREVOGEL, M.J.: CURRENT PHARMACEUTICAL DESIGN, vol. 9, 1 May 2003 (2003-05-01), pages 1033-1040,**
- **CUI, X. ET AL.: J.CLIN.INVEST., vol. 110, no. 4, 2002, pages 515-526,**
- **TYAGI, S.C. ET AL.: MOL. CELL. BIOCHEM., vol. 168, no. 1-2, 1997, pages 1-12,**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### Technical field of the invention

The present invention is in the field of molecular biology, more particularly, the present invention relates to nucleic acid sequences and amino acid sequences of a human PILS and its regulation for the treatment of cardiovascular disorders, endocrine system and hormone disorders, metabolic diseases, gastrointestinal and liver diseases, inflammatory diseases, hematological disorders, respiratory diseases, neurological disorders and urological disorders in mammals.

### Background of the invention

Proteases play a role in carefully controlled processes, such as blood coagulation, fibrinolysis, complement activation, fertilization, and hormone production. These enzymes are also used in a variety of diagnostic, therapeutic, and industrial contexts. PILS is a member of the group of protease enzymes Hattori et al. (1999), Hattori et al. (2000), Saric et al. (2002), York et al. (2002), Akada et al. (2002), WO 200164856, WO 200190304, WO 200122920, WO 200073454.

Proteases were recognized very early in the history of biochemistry. In the nineteenth century, one primary focus of research was on digestive proteases, like pepsin and trypsin. Proteases belong systematically to the C-N Hydrolases. More specifically, proteases catalyze the hyprolytic cleavage of a peptide bond and are therefore called peptidases as well.

Proteases can be classified according to several criteria, e.g. by localisation. Digestive proteases are located in the gastro-intestinal tract. These proteases are responsible for the digestion of food proteins.

Peptidases located extracellularly in the blood or other extracellular compartments of the body play often regulatory roles in processes like for example blood clotting, fibrinolysis, or activation of complement constituents.

Intracellularly located proteases exhibit a wide variety of roles. They are found in compartiments like the ER, the Golgi apparatus, or the lysomes. Their functions include for example activation of peptide hormons, ubiquitin mediated proteolysis, among others.

Proteases are most commonly classified according to their mechanism of action, or to specific active groups that are present in the active center. The following groups can be distinguished:

1. Serin-peptidases, 2. cystein-peptidases, 3. aspartyl- or acidic-peptidases, 4. metallo-peptidases, or 5. peptidases with yet unclear reaction mechanism.

*Serine peptidases*

Serine proteases exhibit a serine in the catalytic site which forms a covalent ester intermediate during the catalytic reaction pathway by a nucleophilic attack on the carboxy carbon of the peptide bond. In the active site of serine proteases a catalytic triad comprised of an aspartate, a histidine and the above mentioned serine is found. This triad functions in the reaction mechanism as a charge relay system.

To the large family of serine protease belong, for example, the digestive enzymes trypsin and chymotrypsin, components of the complement cascade, enzymes involved in the blood clotting cascade, as well as enzymes that function in degradation, rebuilding and maintenace of constituents of the extracellular matrix.

One feature of the serine protease family is the broad range of substrate specificity. Members of the trypase subgroup cleave after arginine or lysine, chymases after phenylalanine or leucine, aspases after aspartate, metases after methionine and serases after serine.

*Cysteine proteases*

During the catalytic reaction of cysteine proteases a covalent thioester intermediate is formed by a nucleophilic attack of the cysteine on the caboxy carbon of the peptide bond. Similar to the serine peptidases a catalytic triad comprised of the cysteine, a histidine and an asparagine is found which functions as a charge relay system to facilitate the formation of the thioester intermediate.

Members of the Cysteine protease family have roles in many different cellular processes, e.g. processing of precursers or intracellular degradation. Examples for cysteine proteases include lysosomal cathepsines, and cytosolic calpains.

*Aspartyl- or acidic peptidase*

The catalytic site of aspartyl proteases is composed of two aspartate residues. At the pH optimum of aspartyl proteases (2-3) one of the aspartyl carboxy groups is ionized and the other is neutral, which is important for the catalytic reaction to occur. Examples for aspartyl proteases are gastric pepsins A and C, chymosin, as well as mammalian renin.

*Metallo-peptidases*

Metallo-peptidases are proteases, whose proteolytic activity depends on the presence of divalent cations in the active center. Examples of members of this class are carboxypeptidase A, which represents a pancreatic digestive enzyme, the Angiotension Converting Enzymes (ACE), which are responsible for the conversion of angiotensin I to angiotensin II, or the Extracellular Matrix Metalloprotienases.

In summary, a huge number of proteases play a central role in several important cellular and intracellular processes. Furthermore, the value as pharmaceutical targets has been proven for several proteases. For example, the protease encoded by the HIV genome is used as a target for drugs for the treatment of HIV infections, the proteasom complex h as b een discovered as an anti-cancer target, or Cys-proteases have been implemented as drug targets for inflammatory disorders. Selective inhibitors have been developed as therapeutic agents for diseases such as HIV. Thus, the identification of further disease implications of protease species and their splice variants may lead to the development of specific inhibitors or modulators, or suggest new utilities for known compounds affecting proteases. That in turn will provide additional pharmacological approaches to treat diseases and conditions in which protease activities are involved. These diseases may include, but are not limited to, infections such as bacterial, fungal, protozoan, and viral infections, particularly those caused by HIV viruses, cancers, allergies including asthma, cardiovascular diseases including acute heart failure, hypotension, hypertension, angina pectoris, myocardial infarction, hematological diseases, genito-urinary diseases including urinary incontinence and benign prostate hyperplasia, osteoporosis, and peripheral and central nervous system disorders including pain, Alzheimer's disease and Parkinson's disease. As regards cardiovascular diseases, Sierevogel et al (2003) disclose matrix metalloproteinases as potential targets for screening drugs, as they are involved in the breakdown of connective tissue in the remodelling phase leading to fibrosis.

**TaqMan-Technology / expression profiling**

TaqMan is a recently developed technique, in which the release of a fluorescent reporter dye from a hybridisation probe in real-time during a polymerase chain reaction (PCR) is proportional to the accumulation of the PCR product. Quantification is based on the early, linear part of the reaction, and by determining the threshold cycle (CT), at which fluorescence above background is first detected.

Gene expression technologies may be useful in several areas of drug discovery and development, such as target identification, lead optimization, and identification of mechanisms of action. The TaqMan technology can be used to compare differences between expression profiles of normal tissue and diseased tissue. Expression profiling has been used in identifying genes, which are up- or downregulated in a variety of diseases. An interesting application of expression profiling is temporal monitoring of changes in gene expression during disease progression and drug treatment or in patients versus healthy individuals. The premise in this approach is that changes in pattern of gene expression in response to physiological or environmental stimuli (e.g., drugs) may serve as indirect clues about disease-causing genes or drug targets. Moreover, the effects of drugs with established efficacy on global gene expression patterns may provide a guidepost, or a genetic signature, against which a new drug candidate can be compared.

**PILS**

The nucleotide sequence of PILS is accessible in public databases by the accession number NM_016442 and is given in SEQ ID NO:1. The amino acid sequence of PILS is depicted in SEQ ID NO:2.

Aminopeptidases play a role in the metabolism of several peptides that may be involved in blood pressure and the pathogenesis of essential hypertension. PILS is a member of the M1 family of zinc metallopeptidases.

By searching an EST database for sequences similar to PILS, Hattori et al. (1999) obtained a full-length cDNA encoding PILS. The deduced 941-amino acid protein contains an N-terminal signal peptide, 5 potential N-glycosylation sites, a potential membrane-spanning domain, and a zinc-binding motif. Northern blot analysis revealed expression of 3.6- and 5.1-kb transcripts in all tissues tested except brain, where only the 5.1-kb mRNA was expressed. An additional 5.6-kb transcript was expressed in spleen and ovary. Hattori et al. (1999) identified a PILS splice variant encoding a protein with 948 residues. Western blot analysis showed expression of an approximately 100-kD cytoplasmic protein, close to the predicted size. Functional analysis indicated a preference for leucine substrates.

By expressing recombinant PILS in Chinese hamster ovary cells, Hattori et al. (2000) showed that PILS is a monomeric protein with a molecular mass of 120 kD that hydrolyzes a variety of bioactive peptides, including angiotensin II. PILS was expressed widely in human tissues, including the cortex of the kidney, where tissue kallikrein is localized. The authors suggested that PILS plays a role in the regulation of blood pressure through inactivation of angiotensin II and/or generation of bradykinin in the kidney.

Saric et al. (2002) and York et al. (2002) determined that purified PILS trims peptides greater than 10 residues in length, but it spares and enhances the production of 8-residue peptides generated either in the ER or cytosol. PILS was also found to trim nearly half of 9-residue peptides, thereby reducing the supply of antigenic peptides in the absence of IFNG expression, which induces PILS expression and instead causes proteasomes to produce more N-terminally extended antigenic precursors and 9-residue peptides.

These results of experiments by Akada et al. (2002) suggest that mPILSAP is involved in the activation of endothelial integrins.

PILS is published in WO 200164856, WO 200190304, WO 200122920 and WO 200073454. PILS shows the highest homology (50%) to the human aminopeptidase as shown in example 1.

The aminopeptidase PILS is described as a type II integral membrane protein with a zinc metalloprotease catalytic motif (Cui et al. 2002). It was shown that the membrane-associated PILS binds in vivo to TNFR1 (TNF receptor 1) and is involved in TNFR1 shedding (Cui et al. 2002). Members of the metalloprotease family are involved in extracellular matrix turnover. (Tyagi, 1997). Both processes are well known mechanism for cardiovascular function.

## Summary of the invention

The invention relates to novel disease associations of PILS polypeptides and polynucleotides. The invention also relates to novel methods of screening for therapeutic agents for the treatment of heart failure, myocardial infarction, ischemic diseases of the heart, arrhythmias, atherosclerosis in the coronary artery or aorta in a mammal. The invention also relates to pharmaceutical compositions for the treatment of heart failure, myocardial infarction, ischemic diseases of the heart, arrhythmias, atherosclerosis in the coronary artery or aorta in a mammal comprising a PILS polypeptide, a PILS polynucleotide, or, an antibody, a ribozyme or an antisense oligonucleotide which regulate PILS polypeptide activity. The invention further comprises methods of diagnosing atherosclerosis in coronary artery or aorta in a mammal.

## Brief Description of the Drawings

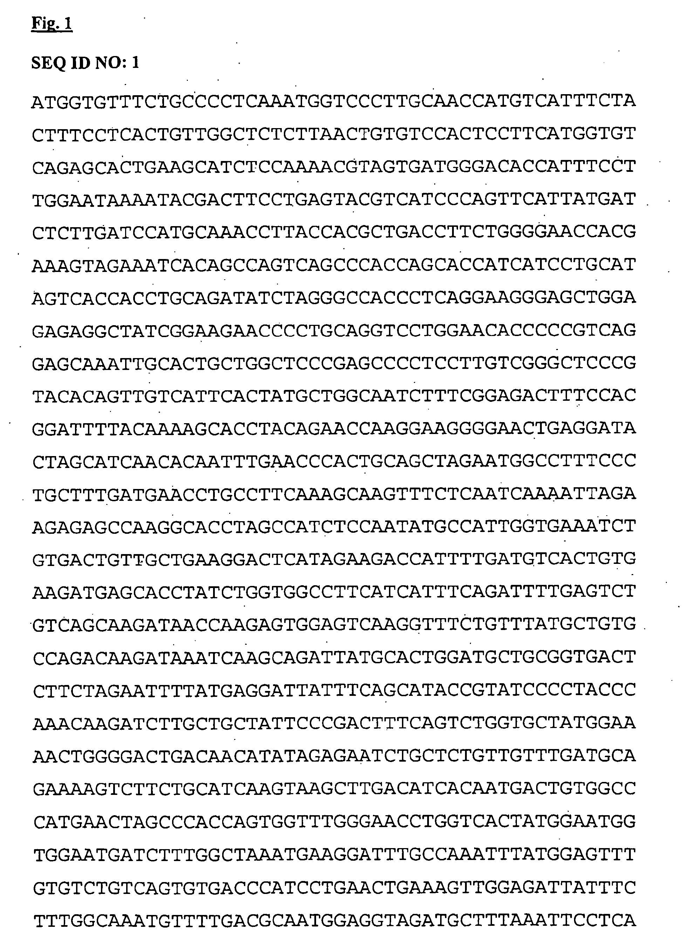

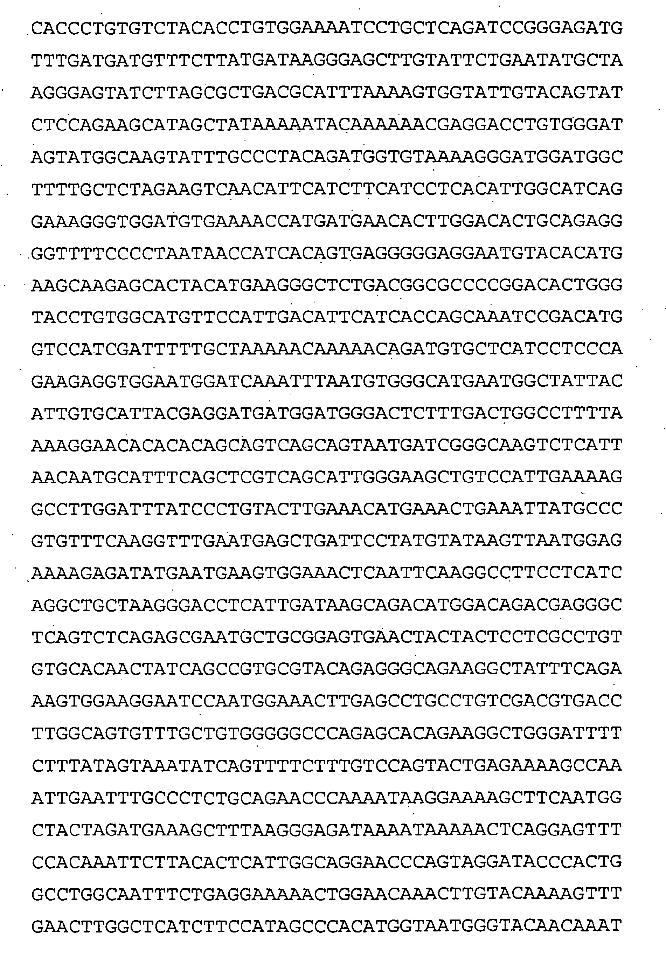

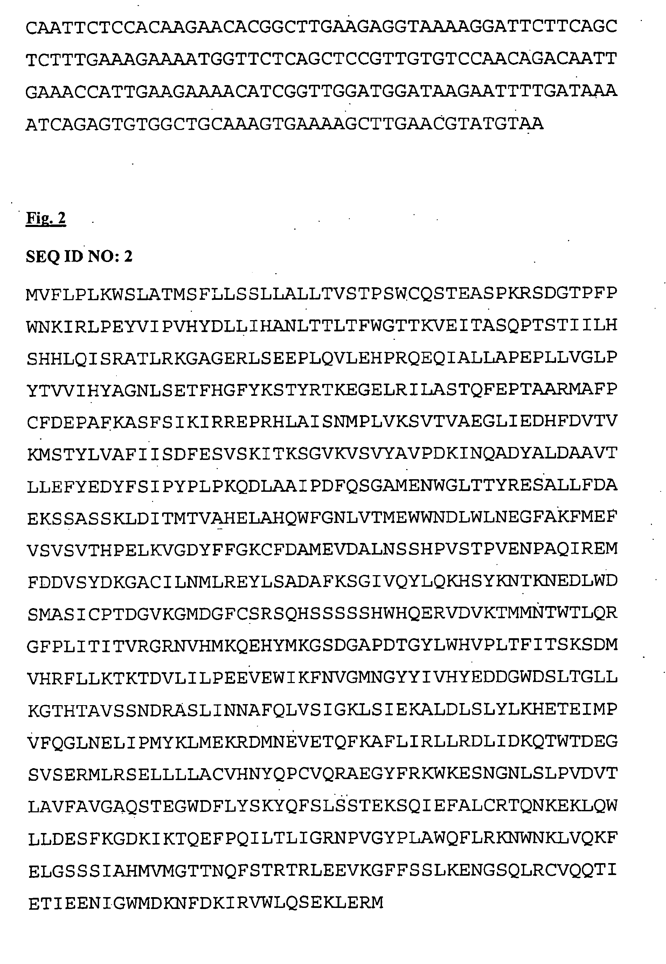

Fig. 1 shows the nucleotide sequence of a PILS polynucleotide (SEQ ID NO:1).
Fig. 2 shows the amino acid sequence of a PILS polypeptide (SEQ ID NO:2).

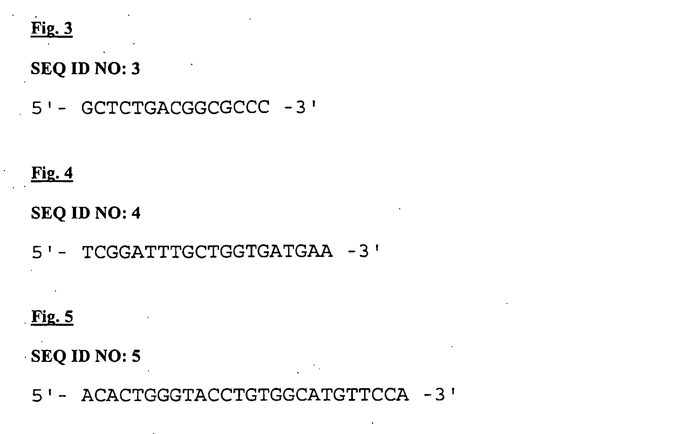

Fig. 3 shows the nucleotide sequence of a primer useful for the invention (SEQ ID NO:3).
Fig. 4 shows the nucleotide sequence of a primer useful for the invention (SEQ ID NO:4).
Fig. 5 shows a nucleotide sequence useful as a probe to detect proteins of the invention (SEQ ID NO:5).

## Detailed description of the invention

### Definition of terms

An "oligonucleotide" is a stretch of nucleotide residues which has a sufficient number of bases to be used as an oligomer, amplimer or probe in a polymerase chain reaction (PCR). Oligonucleotides are prepared from genomic or cDNA sequence and are used to amplify, reveal, or confirm the presence of a similar DNA or RNA in a particular cell or tissue. Oligonucleotides or oligomers comprise portions of a DNA sequence having at least about 10 nucleotides and as many as about 35 nucleotides, preferably about 25 nucleotides.

"Probes" may be derived from naturally occurring or recombinant single- or double-stranded nucleic acids or may be chemically synthesized. They are useful in detecting the presence of identical or similar sequences. Such probes may be labeled with reporter molecules using nick translation, Klenow fill-in reaction, PCR or other methods well known in the art. Nucleic acid probes may be used in southern, northern or in situ hybridizations to determine whether DNA or RNA encoding a certain protein is present in a cell type, tissue, or organ.

A "fragment of a polynucleotide" is a nucleic acid that comprises all or any part of a given nucleotide molecule, the fragment having fewer nucleotides than about 6 kb, preferably fewer than about 1 kb.

"Reporter molecules" are radionuclides, enzymes, fluorescent, chemiluminescent, or chromogenic agents which associate with a particular nucleotide or amino acid sequence, thereby establishing the presence of a certain sequence, or allowing for the quantification of a certain sequence.

"Chimeric" molecules may be constructed by introducing all or part of the nucleotide sequence used in this invention into a vector containing additional nucleic acid sequence which might be expected to change any one or several of the following PILS characteristics: cellular location, distribution, ligand-binding affinities, interchain affinities, degradation/turnover rate, signaling, etc.

"Active", with respect to a PILS polypeptide, refers to those forms, fragments, or domains of a PILS polypeptide which retain the biological and/or antigenic activity of a PILS polypeptide.

"Naturally occurring PILS polypeptide" refers to a polypeptide produced by cells which have not been genetically engineered and specifically contemplates various polypeptides arising from post-translational modifications of the polypeptide including but not limited to acetylation, carboxylation, glycosylation, phosphorylation, lipidation and acylation.

"Derivative" refers to polypeptides which have been chemically modified by techniques such as ubiquitination, labeling (see above), pegylation (derivatization with polyethylene glycol), and chemical insertion or substitution of amino acids such as ornithine which do not normally occur in human proteins.

"Conservative amino acid substitutions" result from replacing one amino acid with another having similar structural and/or chemical properties, such as the replacement of a leucine with an isoleucine or valine, an aspartate with a glutamate, or a threonine with a serine.

"Insertions" or "deletions" are typically in the range of about 1 to 5 amino acids. The variation allowed may be experimentally determined by producing the peptide synthetically while systematically making insertions, deletions, or substitutions of nucleotides in the sequence using recombinant DNA techniques.

A "signal sequence" or "leader sequence" can be used, when desired, to direct the polypeptide through a membrane of a cell. Such a sequence may be naturally present on the polypeptides used in the present invention or provided from heterologous sources by recombinant DNA techniques.

An "oligopeptide" i s a short stretch of amino acid residues and may be expressed from an oligonucleotide. Oligopeptides comprise a stretch of amino acid residues of at least 3, 5, 10 amino acids and at most 10, 15, 25 amino acids, typically of at least 9 to 13 amino acids, and of sufficient length to display biological and/or antigenic activity.

"Inhibitor" i s any substance which retards or prevents a chemical or physiological reaction or response. Common inhibitors include but are not limited to antisense molecules, antibodies, and antagonists.

"Standard expression" is a quantitative or qualitative measurement for comparison. It is based on a statistically appropriate number of normal samples and is created to use as a basis of comparison when performing diagnostic assays, running clinical trials, or following patient treatment profiles.

"Animal" as used herein may be defined to include human, domestic (e.g., cats, dogs, etc.), agricultural (e.g., cows, horses, sheep, etc.) or test species (e.g., mouse, rat, rabbit, etc.).

A "PILS polynucleotide", used in the invention, shall be understood as being a nucleic acid molecule selected from a group consisting of

(i) nucleic acid molecules encoding a polypeptide comprising the amino acid sequence of SEQ ID NO: 2,

(ii) nucleic acid molecules comprising the sequence of SEQ ID NO: 1,

(iii) nucleic acid molecules having the sequence of SEQ ID NO: 1,

(iv) nucleic acid molecules the complementary strand of which hybridizes under stringent conditions to a nucleic acid molecule of (i), (ii), or (iii); and

(v) nucleic acid molecules the sequence of which differs from the sequence of a nucleic acid molecule of (iii) due to the degeneracy of the genetic code;

wherein the polypeptide encoded by said nucleic acid molecule has PILS activity.

A "PILS polypeptide", used in the invention, shall be understood as being a polypeptide selected from a group consisting of

(i) polypeptides having the sequence of SEQ ID NO: 2,

(ii) polypeptides comprising the sequence of SEQ ID NO: 2,

(iii) polypeptides encoded by PILS polynucleotides; and

(iv) polypeptides which show at least 99%, 98%, 95%, 90%, or 80% homology with a polypeptide of (i), (ii), or (iii);

wherein said polypeptide has PILS activity.

The nucleotide sequences encoding a PILS (or their complement) have numerous applications in techniques known to those skilled in the art of molecular biology. These techniques include use as hybridization probes, use in the construction of oligomers for PCR, use for chromosome and gene mapping, use in the recombinant production of PILS, and use in generation of antisense DNA or RNA, their chemical analogs and the like. Uses of nucleotides encoding a PILS disclosed herein are exemplary of known techniques and are not intended to limit their use in any technique known to a person of ordinary skill in the art. Furthermore, the nucleotide sequences disclosed herein may be used in molecular biology techniques that have not yet been developed, provided the new techniques rely on properties of nucleotide sequences that are currently known, e.g., the triplet genetic code, specific base pair interactions, etc.

It will be appreciated by those skilled in the art that as a result of the degeneracy of the genetic code, a multitude of PILS - encoding nucleotide sequences may be produced. Some of these will only bear minimal homology to the nucleotide sequence of the known and naturally occurring PILS. The disclosure has specifically contemplated each and every possible variation of nucleotide sequence that could be made by selecting combinations based on possible codon choices. These combinations are made in accordance with the standard triplet genetic code as applied to the nucleotide sequence of naturally occurring PILS, and all such variations are to be considered as being specifically disclosed.

Although the nucleotide sequences which encode a PILS, its derivatives or its variants are preferably capable of hybridizing to the nucleotide sequence of the naturally occurring PILS polynucleotide under stringent conditions, it may be advantageous to produce nucleotide sequences encoding PILS polypeptides or its derivatives possessing a substantially different codon usage. Codons can be selected to increase the rate at which expression of the peptide occurs in a particular prokaryotic or eukaryotic expression host in accordance with the frequency with which particular codons are utilized by the host. Other reasons for substantially altering the nucleotide sequence encoding a PILS polypeptide and/or its derivatives without altering the encoded a mino acid sequence include the production of RNA transcripts having more desirable properties, such as a greater half-life, than transcripts produced from the naturally occurring sequence.

Nucleotide sequences encoding a PILS polypeptide may be joined to a variety of other nucleotide sequences by means of well established recombinant DNA techniques. Useful nucleotide sequences for joining to PILS polynucleotides include an assortment of cloning vectors such as plasmids, cosmids, lambda phage derivatives, phagemids, and the like. Vectors of interest include expression vectors, replication vectors, probe generation vectors, sequencing vectors, etc. In general, vectors of interest may contain an origin of replication functional in at least one organism, convenient restriction endonuclease sensitive sites, and selectable markers for one or more host cell systems.

Another aspect of the subject is to provide for PILS-specific hybridization probes capable of hybridizing with naturally occurring nucleotide sequences encoding PILS. Such probes may also be used for the detection of similar protease encoding sequences and should preferably show at least 40% nucleotide identity to PILS polynucleotides. The hybridization probes of the subject may be derived from the nucleotide sequence presented as SEQ ID NO: 1 or from genomic sequences including promoter, enhancers or introns of the native gene. Hybridization probes may be labelled by a variety of reporter molecules using techniques well known in the art.

It will be recognized that many deletional or mutational analogs of PILS polynucleotides will be effective hybridization probes for PILS polynucleotides. Accordingly, the invention relates to nucleic acid sequences that hybridize with such PILS encoding nucleic acid sequences under stringent conditions.

"Stringent conditions" refers to conditions that allow for the hybridization of substantially related nucleic acid sequences. For instance, such conditions will generally allow hybridization of sequence with at least about 85% sequence identity, preferably with at least about 90% sequence identity, more preferably with at least about 95% sequence identity. Hybridization conditions and probes can be adjusted in well-characterized ways to achieve selective hybridization of human-derived probes. Stringent conditions, within the meaning of the invention are 65°C in a buffer containing 1 mM EDTA, 0.5 M $NaHPO_4$ (pH 7.2), 7 % (w/v) SDS.

Nucleic acid molecules that will hybridize to PILS polynucleotides under stringent conditions can be identified functionally. Without limitation, examples of the uses for hybridization probes include: histochemical uses such as identifying tissues that express PILS; measuring mRNA levels, for instance to identify a sample's tissue type or to identify cells that express abnormal levels of PILS; and detecting polymorphisms of PILS.

PCR. provides additional uses for oligonucleotides based upon the nucleotide sequence which encodes PILS. Such probes used in PCR may be of recombinant origin, chemically synthesized, or a mixture of both. Oligomers may comprise discrete nucleotide sequences employed under optimized conditions for identification of PILS in specific tissues or diagnostic use. The same two oligomers, a nested set of oligomers, or even a degenerate pool of oligomers may be employed under less stringent conditions for identification of closely related DNAs or RNAs.

Rules for designing polymerase chain reaction (PCR) primers are now established, as reviewed by PCR Protocols. Degenerate primers, i.e., preparations of primers that are heterogeneous at given sequence locations, can be designed to amplify nucleic acid sequences that are highly homologous to, but not identical with PILS. Strategies are now available that allow for only one of the primers to be required to specifically hybridize with a known sequence. For example, appropriate nucleic acid primers can be ligated to the nucleic acid sought to be amplified to provide the hybridization partner for one of the primers. In this way, only one of the primers need be based on the sequence of the nucleic acid sought to be amplified.

PCR methods for amplifying nucleic acid will utilize at least two primers. One of these primers will be capable of hybridizing to a first strand of the nucleic acid to be amplified and of priming enzyme-driven nucleic acid synthesis in a first direction. The other will be capable of hybridizing the reciprocal sequence of the first strand (if the sequence to be amplified is single stranded, this sequence will initially be hypothetical, but will be synthesized in the first amplification cycle) and of priming nucleic acid synthesis from that strand in the direction opposite the first direction and towards the site of hybridization for the first primer. Conditions for conducting such amplifications, particularly under preferred stringent hybridization conditions, are well known.

Other means of producing specific hybridization probes for PILS include the cloning of nucleic acid sequences encoding PILS or PILS derivatives into vectors for the production of mRNA probes. Such vectors are known in the art, are commercially available and may be used to synthesize RNA probes in vitro by means of the addition of the appropriate RNA polymerase as T7 or SP6 RNA polymerase and the appropriate reporter molecules.

It is possible to produce a DNA sequence, or portions thereof, entirely by synthetic chemistry. After synthesis, the nucleic acid sequence can be inserted into any of the many available DNA vectors and their respective host cells using techniques which are well known in the art. Moreover, synthetic chemistry may be used to introduce mutations into the nucleotide sequence. Alternately, a portion of sequence in which a mutation is desired can be synthesized and recombined with longer portion of an existing genomic or recombinant sequence.

PILS polynucleotides may be used to produce a purified oligo-or polypeptide using well known methods of recombinant DNA technology. The oligopeptide may be expressed in a variety of host cells, either prokaryotic or eukaryotic. Host cells may be from the same species from which the nucleotide sequence was derived or from a different species. Advantages of producing an oligonucleotide by recombinant DNA technology include obtaining adequate amounts of the protein for purification and the availability of simplified purification procedures.

**Quantitative determinations of nucleic acids**

An important step in the molecular genetic analysis of human disease is often the enumeration of the copy number of a nucleis acid or the relative expression of a gene in particular tissues.

Several different approaches are currently available to make quantitative determinations of nucleic acids. Chromosome-based techniques, such as comparative genomic hybridization (CGH) and fluorescent in situ hybridization (FISH) facilitate efforts to cytogenetically localize genomic regions that are altered in tumor cells. Regions of-genomic alteration can be narrowed further using loss of heterozygosity analysis (LOH), in which disease DNA is analyzed and compared with normal DNA for the loss of a heterozygous polymorphic marker. The first experiments used restriction fragment length polymorphisms (RFLPs) [Johnson, (1989)], or hyper-variable minisatellite DNA [Barnes, 2000]. In recent years LOH has been performed primarily using PCR amplification of microsatellite markers and electrophoresis of the radio labelled [Jeffreys, (1985)] or fluorescently labelled PCR products [Weber, (1990)] and compared between paired normal and disease DNAs.

A number of other methods have also been developed to quantify nucleic acids [Gergen, (1992)]. More recently, PCR and RT-PCR methods have been developed which are capable of measuring the amount of a nucleic acid in a sample. One approach, for example, measures PCR product quantity in the log phase of the reaction before the formation of reaction products plateaus [Thomas, (1980)].

A gene sequence contained in all samples at relatively constant quantity is typically utilized for sample amplification efficiency normalization. This approach, however, suffers from several drawbacks. The method requires t hat e ach sample h as e qual input amounts of the nucleic acid and that the amplification efficiency between samples is identical until the time of analysis. Furthermore, it is difficult using the conventional methods of PCR quantitation such as gel electrophoresis or plate capture hybridization to determine that all samples are in fact analyzed during the log phase of the reaction as required by the method.

Another method called quantitative competitive (QC)-PCR, as the name implies, relies on the inclusion of an internal control competitor in each reaction [Piatak, (1993), BioTechniques]. The efficiency of each reaction is normalized to the internal competitor. A known amount of internal competitor is typically added to each sample. The unknown target PCR product is compared with the known competitor PCR product to obtain relative quantitation. A difficulty with this general approach lies in developing an internal control that amplifies with the same efficiency than the target molecules.

*5' Fluorogenic Nuclease Assays*

Fluorogenic nuclease assays are a real time quantitation method that uses a probe to monitor formation of amplification product. The basis for this method of monitoring the formation of amplification product is to measure continuously PCR product accumulation u sing a d ual-labelled fluorogenic oligonucleotide probe, an approach frequently referred to in the literature simply as the "TaqMan method" [Piatak,(1993), Science; Heid, (1996); Gibson, (1996); Holland. (1991)].

The probe used in such assays is typically a short (about 20-25 bases) oligonucleotide that is labeled with two different fluorescent dyes. The 5' terminus of the probe is attached to a reporter dye and the 3' terminus is attached to a quenching dye, although the dyes could be attached at other locations on the probe as well. The probe is designed to have at least substantial sequence complementarity with the probe binding site. Upstream and downstream PCR primers which bind to flanking regions of the locus are added to the reaction mixture. When the probe is intact, energy transfer between the two fluorophors occurs and the quencher quenches emission from the reporter. During the extension phase of PCR, the probe is cleaved by the 5' nuclease activity of a nucleic acid polymerase such as Taq polymerase, thereby releasing the reporter from the oligonucleotide-quencher and resulting in an increase of reporter emission intensity which can be measured by an appropriate detector.

One detector which is specifically adapted for measuring fluorescence emissions such as those created during a fluorogenic assay is the ABI 7700 or 4700 HT manufactured by Applied Biosystems, Inc. in Foster City, Calif. The ABI 7700 uses fiber o ptics c onnected w ith e ach well i n a 9 6-or 3 84 w ell P CR tube arrangement. The instrument includes a laser for exciting the labels and is capable of measuring the fluorescence spectra intensity from each tube with continuous monitoring during PCR amplification. Each tube is re-examined every 8.5 seconds.

Computer software provided with the instrument is capable of recording the fluorescence intensity of reporter and quencher over the course of the amplification. The recorded values will then be used to calculate the increase in normalized reporter emission intensity on a continuous basis. The increase in emission intensity is plotted versus time, i.e., the number of amplification cycles, to produce a continuous measure of amplification. To quantify the locus in each amplification reaction, the amplification plot is examined at a point during the log phase of product accumulation. This is accomplished by assigning a fluorescence threshold intensity above background and determining the point at which each amplification plot crosses the threshold (defined as the threshold cycle number or Ct). Differences in threshold cycle number are used to quantify the relative amount of PCR target contained within each tube. Assuming that each reaction functions at 100% PCR efficiency, a difference of one Ct represents a two-fold difference in the amount of starting template. The fluorescence value can be used in conjunction with a standard curve to determine the amount of amplification product present.

*Non-Probe-Based Detection Methods*

A variety of options are available for measuring the amplification products as they are formed. One method utilizes labels, such as dyes, which only b ind to double stranded DNA. In this type of approach, amplification product (which is double stranded) binds dye molecules in solution to form a complex. With the appropriate dyes, it is possible to distinguish between dye molecules free in solution and dye molecules bound to amplification product. For example, certain dyes fluoresce only when bound to amplification product. Examples of dyes which can be used in methods of this general type include, but are not limited to, Syber Green.TM. and Pico Green from Molecular Probes, Inc. of Eugene, Oreg., ethidium bromide, propidium i odide, c hromomycin, acridine orange, Hoechst 33258, Toto-1, Yoyo-1, DAPI (4', 6-diamidino-2-phenylindole hydrochloride).

Another real time detection technique measures alteration in energy fluorescence energy transfer between fluorophors conjugated with PCR primers [Livak, (1995)].

*Probe-Based Detection Methods*

These detection methods involve some alteration to the structure or conformation of a probe hybridized to the locus between the amplification primer pair. In some instances, the alteration is caused by the template-dependent extension catalyzed by a nucleic acid polymerase during the amplification process. The alteration generates a detectable signal which is an indirect measure of the amount of amplification product formed.

For example, some methods involve the degradation or digestion of the probe during the extension reaction. These methods are a consequence of the 5'-3' nuclease activity associated with some nucleic acid polymerases. Polymerases having this activity cleave mononucleotides or small oligonucleotides from a n oligonucleotide probe annealed to its complementary sequence located within the locus.

The 3' end of the upstream primer provides the initial binding site for the nucleic acid polymerase. As the polymerase catalyzes extension of the upstream primer and encounters the bound probe, the nucleic acid polymerase displaces a portion of the 5' end of the probe and through its nuclease activity cleaves mononucleotides or oligonucleotides from the probe.

The upstream primer and the probe can be designed such that they anneal to the complementary strand in close proximity to one another. In fact, the 3' end of the upstream primer and the 5' end of the probe may abut one another. In this situation, extension of the upstream primer is not necessary in order for the nucleic acid polymerase to begin cleaving the probe. In the case in which intervening nucleotides separate the upstream primer and the probe, extension of the primer is necessary before the nucleic acid polymerase encounters the 5' end of the probe. Once contact occurs and polymerization continues, the 5'-3' exonuclease activity of the nucleic acid polymerase begins cleaving mononucleotides or oligonucleotides from the 5' end of the probe. Digestion of the probe continues until the remaining portion of the probe dissociates from the complementary strand.

In solution, the two end sections can hybridize with each other to form a hairpin loop. In this conformation, the reporter and quencher dye are in sufficiently close proximity that fluorescence from the reporter dye is effectively quenched by the quencher dye. Hybridized probe, in contrast, results in a linearized conformation in which the extent of quenching is decreased. Thus, by monitoring emission changes for the two dyes, it is possible to indirectly monitor the formation of amplification product.

*Probes*

The labeled probe is selected so that its sequence is substantially complementary to a segment of the test locus or a reference locus. As indicated above, the nucleic acid site to which the probe binds should be located between the primer binding sites for the upstream and downstream amplification primers.

*Primers*

The primers used in the amplification are selected so as to be capable of hybridizing to sequences at flanking regions of the locus being amplified. The primers are chosen to have at least substantial complementarity with the different strands of the nucleic acid being amplified. When a probe is utilized to detect the formation of amplification products, the primers are selected in such that they flank the probe, i.e. are located upstream and downstream of the probe.

The primer must have sufficient length so that it is capable of priming the synthesis of extension products in the presence of an agent for polymerization. The length and composition of the primer depends on many parameters, including, for example, the temperature at which the annealing reaction is conducted, proximity of the probe binding site to that of the primer, relative concentrations of the primer and probe and the particular nucleic acid composition of the probe. Typically the primer includes 15-30 nucleotides. However, the length of the primer may be more or less depending on the complexity of the primer binding site and the factors listed above.

*Labels for Probes and Primers*

The labels used for labeling the probes or primers of the current disclosure and which can provide the signal corresponding to the quantity of amplification product can take a variety of forms. As indicated above with regard to the 5' fluorogenic nuclease method, a fluorescent signal is one signal which can be measured. However, measurements may also be made, for example, by monitoring radioactivity, colorimetry, absorption, magnetic parameters, or enzymatic activity. Thus, labels which can be employed include, but are not limited to, fluorophors, chromophores, radioactive isotopes, electron dense reagents, enzymes, and ligands having specific binding partners (e.g., biotin-avidin).

Monitoring changes in fluorescence is a particularly useful way to monitor the accumulation of amplification products. A number of labels useful for attachment to probes or primers are commercially available including fluorescein and various fluorescein derivatives such as FAM, HEX, TET and JOE (all which are available from Applied Biosystems, Foster City, Calif.); lucifer yellow, and coumarin derivatives.

Labels may be attached to the probe or primer using a variety of techniques and can be attached at the 5' end, and/or the 3' end and/or at an internal nucleotide. The label can also be attached to spacer arms of various sizes which are attached to the probe or p rimer. T hese s pacer a rms a re useful for obtaining a desired distance between multiple labels attached to the probe or primer.

In some instances, a single label may be utilized; whereas, in other instances, such as with the 5' fluorogenic nuclease assays for example, two or more labels are attached to the probe. In cases wherein the probe includes multiple labels, it is generally advisable to maintain spacing between the labels which is sufficient to permit separation of the labels during digestion of the probe through the 5'-3' nuclease activity of the nucleic acid polymerase.

**Patients Exhibiting Symptoms of Disease**

A number of diseases are associated with changes in the copy number of a certain gene. For patients having symptoms of a disease, the real-time PCR method can be used to determine if the patient has copy number alterations which are known to be linked with diseases that are associated with the symptoms the patient has.

**PILS expression**

*PILS fusion proteins*

Fusion proteins are useful for generating antibodies against PILS polypeptides and for use in various assay systems. For example, fusion proteins can be used to identify proteins which interact with portions of PILS polypeptides. Protein affinity chromatography or library-based assays for protein-protein interactions, such as the yeast two-hybrid or phage display systems, can be used for this purpose. Such methods are well known in the art and also can be used as drug screens.

A PILS fusion protein comprises two polypeptide segments fused together by means of a peptide bond. The first polypeptide segment can comprise at least 54, 75, 100, 125, 139, 150, 175, 200, 225, 250, 275, 300, 325 or 350 contiguous amino acids of SEQ ID NO: 2 or of a biologically active variant, such as those described above. The first polypeptide segment also can comprise full-length PILS.

The second polypeptide segment can be a full-length protein or a protein fragment. Proteins commonly used in fusion protein construction include, but are not limited to β-galactosidase, β-glucuronidase, green fluorescent protein (GFP), autofluorescent proteins, including blue fluorescent protein (BFP), glutathione-S-transferase (GST), luciferase, horseradish peroxidase (HRP), and chloramphenicol acetyltransferase (CAT). Additionally, epitope tags are used in fusion protein constructions, including histidine (His) tags, FLAG tags, influenza hemagglutinin (HA) tags, Myc tags, VSV-G tags, and thioredoxin (Trx) tags. Other fusion constructions can include maltose binding protein (MBP), S-tag, L ex a D NA b inding d omain (DBD) fusions, GAL4 DNA binding domain fusions, and herpes simplex virus (HSV) BP16 protein fusions. A fusion protein also can be engineered to contain a cleavage site located adjacent to the PILS.

*Preparation of Polynucleotides*

A naturally occurring PILS polynucleotide can be isolated free of other cellular components such as membrane components, proteins, and lipids. Polynucleotides can be made by a cell and isolated using standard nucleic acid purification techniques, or synthesized using an amplification technique, such as the polymerase chain reaction (PCR), or by using an automatic synthesizer. Methods for isolating polynucleotides are routine and are known in the art. Any such technique for obtaining a polynucleotide can be used to obtain isolated PILS polynucleotides. For example, restriction enzymes and probes can be used to isolate polynucleotide fragments which comprise PILS nucleotide sequences. Isolated polynucleotides are in preparations which are free or at least 70, 80, or 90% free of other molecules.

PILS cDNA molecules can be made with standard molecular biology techniques, using PILS mRNA as a template. PILS cDNA molecules can thereafter be replicated using molecular biology techniques known in the art. An amplification technique, such as PCR, can be used to obtain additional copies of polynucleotides used in the invention, using either human genomic DNA or cDNA as a template.

Alternatively, synthetic chemistry techniques can be used to synthesizes PILS polynucleotides. The degeneracy of the genetic code allows alternate nucleotide sequences to be synthesized which will encode PILS having, for example, an amino acid sequence shown in SEQ ID NO: 2 or a biologically active variant thereof.

*Extending Polynucleotides*

Various PCR-based methods can be used to extend nucleic acid sequences encoding human PILS, for example to detect upstream sequences of PILS gene such as promoters and regulatory elements. For example, restriction-site PCR uses universal primers to retrieve unknown sequence adjacent to a known locus. Genomic DNA is first amplified in the presence of a primer to a linker sequence and a primer specific to the known region. The amplified sequences are then subjected to a second round of PCR with the same linker primer and another specific primer internal to the first one. Products of each round of PCR are transcribed with an appropriate RNA polymerase and sequenced using reverse transcriptase.

Inverse PCR also can be used to amplify or extend sequences using divergent primers based on a known region. P rimers c an b e d esigned u sing commercially available software, such as OLIGO 4.06 Primer Analysis software (National Biosciences Inc., Plymouth, Minn.), to be 22-30 nucleotides in length, to have a GC content of 50% or more, and to anneal to the target sequence at temperatures about 68-72°C. The method uses several restriction enzymes to generate a suitable fragment in the known region of a gene. The fragment is then circularized by intramolecular ligation and used as a PCR template.

Another method which can be used is capture PCR, which involves PCR amplification of DNA fragments adjacent to a known sequence in human and yeast artificial chromosome DNA. In this method, multiple restriction enzyme digestions and ligations also can be used to place an engineered double-stranded sequence into an unknown fragment of the DNA molecule before performing PCR.

When screening for full-length cDNAs, it is preferable to use libraries that have been size-selected to include larger cDNAs. Randomly-primed libraries are preferable, in that they will contain more sequences which contain the 5' regions of genes. Use of a randomly primed library may be especially preferable for situations in which an oligo d(T) library does not yield a full-length cDNA. Genomic libraries can be useful for extension of sequence into 5' non-transcribed regulatory regions.

Commercially available capillary electrophoresis systems can be used to analyze the size or confirm the nucleotide sequence of PCR or sequencing products. For example, capillary sequencing can employ flowable polymers for electrophoretic separation, four different fluorescent dyes (one for each nucleotide) which are laser activated, and detection of the emitted wavelengths by a charge coupled device camera. Output/light intensity can be converted to electrical signal using appropriate equipment and software (e.g., G ENOTYPER and Sequence NAVIGATOR, Perkin Elmer), and the entire process from loading of samples to computer analysis and electronic data display can be computer controlled. Capillary electrophoresis is especially preferable for the sequencing of small pieces of DNA which might be present in limited amounts in a particular sample.

*Obtaining Polypeptides*

PILS can be obtained, for example, by purification from human cells, by expression of PILS polynucleotides, or by direct chemical synthesis.

*Protein Purification*

PILS can be purified from any human cell which expresses the enzyme, including those which have been transfected with expression constructs which express PILS. A purified PILS is separated from other compounds which normally associate with PILS in the cell, such as certain proteins, carbohydrates, or lipids, using methods well-known in the art. Such methods include, but are not limited to, size exclusion chromatography, ammonium sulfate fractionation, ion exchange chromatography, affinity chromatography, and preparative gel electrophoresis.

*Expression of PILS Polynucleotides*

To express PILS, PILS polynucleotides can be inserted into an expression vector which contains the necessary elements for the transcription and translation of the inserted coding sequence. Methods which are well known to those skilled in the art can be used to construct expression vectors containing sequences encoding PILS and appropriate transcriptional and translational control elements. These methods include *in vitro* recombinant DNA techniques, synthetic techniques, and *in vivo* genetic recombination.

A variety of expression vector/host systems can be utilized to contain and express sequences encoding P ILS. T hese i nclude, b ut a re n ot limited t o, m icroorganisms, such as bacteria transformed with recombinant bacteriophage, plasmid, or cosmid DNA expression vectors; yeast transformed with yeast expression vectors, insect cell systems infected with virus expression vectors (e.g., baculovirus), plant cell systems transformed with virus expression vectors (e.g., cauliflower mosaic virus, CaMV; tobacco mosaic virus, TMV) or with bacterial expression vectors (e.g., Ti or pBR322 plasmids), or animal cell systems.

The control elements or regulatory sequences are those non-translated regions of the vector - enhancers, promoters, 5' and 3' untranslated regions -- which interact with host cellular proteins to carry out transcription and translation. Such elements can vary in their strength and specificity. Depending on the vector system and host utilized, any number of suitable transcription and translation elements, including constitutive and inducible promoters, can be used. F or example, when cloning in bacterial systems, inducible promoters such as the hybrid lacZ promoter of the BLUESCRIPT phagemid (Stratagene, LaJolla, Calif.) or pSPORT1 plasmid (Life Technologies) and the like can be used. The baculovirus polyhedrin promoter can be used in insect cells. Promoters or enhancers derived from the genomes of plant cells (*e.g.,* heat shock, RUBISCO, and storage protein genes) or from plant viruses (*e.g.,* viral promoters or leader sequences) can be cloned into the vector. In mammalian cell systems, promoters from mammalian genes or from mammalian viruses are preferable. If it is necessary to generate a cell line that contains multiple copies of a nucleotide sequence encoding PILS, vectors based on SV40 or EBV can be used with an appropriate selectable marker.

*Bacterial and Yeast Expression Systems*

In bacterial systems, a number of expression vectors can be selected. For example, when a large quantity of PILS is needed for the induction of antibodies, vectors which direct high level expression of fusion proteins that are readily purified can be used. Such vectors include, but are not limited to, multifunctional *E. coli* cloning and expression vectors such as BLUESCRIPT (Stratagene). In a BLUESCRIPT vector, a sequence encoding PILS can be ligated into the vector in frame with sequences for the amino-terminal Met and the subsequent 7 residues of β-galactosidase so that a hybrid protein is produced. pIN vectors or pGEX vectors (Promega, Madison, Wis.) also can be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems can be designed to include heparin, thrombin, or factor Xa protease cleavage sites so that the cloned polypeptide of interest can be released from the GST moiety at will.

*Plant and Insect Expression Systems*

If plant expression vectors are used, the expression of sequences encoding PILS can be driven by any of a number of promoters. For example, viral promoters such as the 35S and 19S promoters of CaMV can be used alone or in combination with the omega leader sequence from TMV. Alternatively, plant promoters such as the small subunit of RUBISCO or heat shock promoters can be used. These constructs can be introduced into plant cells by direct DNA transformation or by pathogen-mediated transfection.

An insect system also can be used to express PILS. For example, in one such system *Autographa californica* nuclear polyhedrosis virus (AcNPV) is used as a vector to express foreign genes in *Spodoptera frugiperda* cells or in *Trichoplusia* larvae. Sequences encoding PILS can be cloned into a non-essential region of the virus, such as the polyhedrin gene, and placed under control of the polyhedrin promoter. Successful insertion of PILS w ill render the polyhedrin gene inactive and produce recombinant virus lacking coat protein. The recombinant viruses can then be used to infect S. *frugiperda cells* or *Trichoplusia* larvae in which PILS can be expressed.

*Mammalian Expression Systems*

A number of viral-based expression systems can be used to express PILS in mammalian host cells. For example, if an adenovirus is used as an expression vector, sequences encoding PILS can be ligated into an adenovirus transcription/translation complex comprising the late promoter and tripartite leader sequence. Insertion in a non-essential E1 or E3 region of the viral genome can be used to obtain a viable virus which is capable of expressing PILS in infected h ost c ells [ Engelhard, 1 994)]. I f desired, transcription enhancers, such as the Rous sarcoma virus (RSV) enhancer, can be used to increase expression in mammalian host cells.

Human artificial chromosomes (HACs) also can be used to deliver larger fragments of DNA than can be contained and expressed in a plasmid. HACs of 6M to 10M are constructed and delivered to cells via conventional delivery methods (e.g., liposomes, polycationic amino polymers, or vesicles). Specific initiation signals also can be used to achieve more efficient translation of sequences encoding PILS. Such signals include the ATG initiation codon and adjacent sequences. In cases where sequences encoding PILS, its initiation codon, and upstream sequences are inserted into the appropriate expression vector, no additional transcriptional or translational control signals may be needed. However, in cases where only coding sequence, or a fragment thereof, is inserted, exogenous translational control signals (including the ATG i nitiation c odon) s hould be provided. The initiation c odon s hould b e in the correct reading frame to ensure translation of the entire insert. Exogenous translational elements and initiation codons can be of various origins, both natural and synthetic.

*Host Cells*

A host cell strain can be chosen for its ability to modulate the expression of the inserted sequences or to process the expressed PILS in the desired fashion. S uch modifications of the polypeptide include, but are not limited to, acetylation, carboxylation, glycosylation, phosphorylation, lipidation, and acylation. Post-translational processing which cleaves a "prepro" form of the polypeptide also can be used to facilitate correct insertion, folding and/or function. Different host cells which have specific cellular machinery and characteristic mechanisms for post-translational activities (e.g., CHO, HeLa, MDCK, HEK293, and WI38), are available from the American Type Culture Collection (ATCC; 10801 University Boulevard, Manassas, VA 20110-2209) and can be chosen to ensure the correct modification and processing of the foreign protein.

Stable expression is preferred for long-term, high-yield production of recombinant proteins. For example, cell lines which stably express PILS can be transformed using expression vectors which can contain viral origins of replication and/or endogenous expression elements and a selectable marker gene on the same or on a separate vector. Following the introduction of the vector, cells can be allowed to grow for 1-2 days in an enriched medium before they are switched to a selective medium. The purpose of the selectable marker is to confer resistance to selection, and its presence allows growth and recovery of cells which successfully express the introduced PILS sequences. Resistant clones of stably transformed cells can be proliferated using tissue culture techniques appropriate to the cell type. Any number of selection systems can be used to recover transformed cell lines. These include, but are not limited to, the herpes simplex virus thymidine kinase [Logan, (1984)] and adenine phosphoribosyltransferase [ Wigler, (1977)] g enes which c an b e employed in *tk*⁻ or *aprt*⁻ cells, respectively. Also, antimetabolite, antibiotic, or herbicide resistance can be used as the basis for selection. For example, *dhfr* confers resistance to methotrexate [Lowy, (1980)], *npt* confers resistance to the aminoglycosides, neomycin and G-418 [Wigler, (1980)], and *als* and *pat* confer resistance to chlorsulfuron and phosphinotricin acetyltransferase, respectively [Colbere-Garapin, 1981]. Additional selectable genes have been described. For example, *trpB* allows cells to utilize indole in place of tryptophan, or *hisD,* which allows cells to utilize histinol in place of histidine. Visible markers such as anthocyanins, β-glucuronidase and its substrate GUS, and luciferase and its substrate luciferin, can be used to identify transformants and to quantify the amount of transient or stable protein expression attributable to a specific vector system

*Detecting Polypeptide Expression*

Although the presence of marker gene expression suggests that a PILS polynucleotide is also present, its presence and expression may need to be confirmed. For example, if a s equence encoding P ILS i s inserted w ithin a m arker g ene s equence, transformed cells containing sequences which encode PILS can be identified by the absence of marker gene function. Alternatively, a marker gene can be placed in tandem with a sequence encoding PILS under the control of a single promoter. Expression of the marker gene in response to induction or selection usually indicates expression of PILS polynucleotide.

Alternatively, host cells which contain a PILS polynucleotide and which express PILS can be identified by a variety of procedures known to those of skill in the art. These procedures include, but are not limited to, DNA-DNA or DNA-RNA hybridizations and protein bioassay or immunoassay techniques which include membrane, solution, or chip-based technologies for the detection and/or quantification of nucleic acid or protein. For example, the presence of a polynucleotide sequence encoding PILS can be detected by DNA-DNA or DNA-RNA hybridization or amplification using probes or fragments or fragments of polynucleotides encoding PILS. Nucleic acid amplification-based assays involve the use of oligonucleotides selected from sequences encoding PILS to detect transformants which contain a PILS polynucleotide.

A variety of protocols for detecting and measuring the expression of PILS, using either polyclonal or monoclonal antibodies specific for the polypeptide, are known in the art. Examples include enzyme-linked immunosorbent assay (ELISA), radioimmunoassay (RIA), and fluorescence activated cell sorting (FACS). A two-site, monoclonal-based immunoassay using monoclonal antibodies reactive to two non-interfering epitopes on PILS can be used, or a competitive binding assay can be employed.

A wide variety of labels and conjugation techniques are known by those skilled in the art and can be used in various nucleic acid and amino acid assays. Means for producing labeled hybridization or PCR probes for detecting sequences related to polynucleotides encoding PILS include oligolabeling, nick translation, end-labeling, or PCR amplification using a labeled nucleotide. Alternatively, sequences encoding PILS can be cloned into a vector for the production of an mRNA probe. Such vectors are known in the art, are commercially available, and can be used to synthesize RNA probes *in vitro* by addition of labeled nucleotides and an appropriate RNA polymerase such as T7, T3, or SP6. These procedures can be conducted using a variety of commercially available kits (Amersham Pharmacia B iotech, P romega, and US Biochemical). Suitable reporter molecules or labels which can be used for ease of detection include radionuclides, enzymes, and fluorescent, chemiluminescent, or chromogenic agents, as well as substrates, cofactors, inhibitors, magnetic particles, and the like.

*Expression and Purification of Polypeptides*

Host cells transformed with PILS polynucleotides can be cultured under conditions suitable for the expression and recovery of the protein from cell culture. The polypeptide produced by a transformed cell can be secreted or contained intracellularly depending on the sequence and/or the vector used. As will be understood by those of skill in the art, expression vectors containing PILS polynucleotides can be designed to contain signal sequences which direct secretion of soluble PILS through a prokaryotic or eukaryotic cell membrane or which direct the membrane insertion of membrane-bound PILS.

As discussed above, other constructions can be used to join a sequence encoding PILS to a nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). Inclusion of cleavable linker sequences such as those specific for Factor XA or enterokinase (Invitrogen, San Diego, CA) between the purification domain and PILS also can be used to facilitate purification. One such expression vector provides for expression of a fusion protein containing PILS and 6 histidine residues preceding a thioredoxin or an enterokinase cleavage site. The histidine residues facilitate purification by IMAC (immobilized metal ion affinity chromatography) Maddox, (1983)], while the enterokinase cleavage site provides a means for purifying PILS from the fusion protein [Porath, (1992)].

*Chemical Synthesis*

Sequences encoding PILS can be synthesized, in whole or in part, using chemical methods well known in the art. Alternatively, PILS itself can be produced using chemical methods to synthesize its amino acid sequence, such as by direct peptide synthesis using solid-phase techniques. Protein synthesis can either be performed using manual techniques or by automation. Automated synthesis can be achieved, for example, using Applied Biosystems 431A Peptide Synthesizer (Perkin E lmer). Optionally, fragments of PILS can be separately synthesized and combined using chemical methods to produce a full-length molecule.

The newly synthesized peptide can be substantially purified by preparative high performance liquid chromatography. The composition of a synthetic PILS can be confirmed by amino acid analysis or sequencing. Additionally, any portion of the amino acid sequence of PILS can be altered during direct synthesis and/or combined using chemical methods with sequences from other proteins to produce a variant polypeptide or a fusion protein.

*Production of Altered Polypeptides*

As will be understood by those of skill in the art, it may be advantageous to produce PILS polynucleotides possessing non-naturally occurring codons. For example, codons preferred by a particular prokaryotic or eukaryotic host can be selected to increase the rate of protein expression or to produce an RNA transcript having desirable properties, such as a half-life which is longer than that of a transcript generated from the naturally occurring sequence.

The nucleotide sequences referred to herein can be engineered using methods generally known in the art to alter PILS polynucleotides for a variety of reasons, including but not limited to, alterations which modify the cloning, processing, and/or expression of the polypeptide or mRNA product. DNA shuffling by random fragmentation and PCR reassembly of gene fragments and synthetic oligonucleotides can be used to engineer the nucleotide sequences. For example, site-directed mutagenesis can be used to insert new restriction sites, alter glycosylation patterns, change codon preference, produce splice variants, introduce mutations, and so forth.

*PILS Analogs*

One general class of PILS analogs are variants having an amino acid sequence that is a mutation of the amino acid sequence disclosed herein. Another general class of PILS analogs is provided by anti-idiotype antibodies, and fragments thereof, as described below. Moreover, recombinant antibodies comprising anti-idiotype variable domains can be used as analogs (see, for example, [Monfardini et al., (1996)]). Since the variable domains of anti-idiotype PILS antibodies mimic PILS, these domains can provide PILS enzymatic activity. Methods of producing anti-idiotypic catalytic antibodies are known to those of skill in the art [Joron et al., (1992), Friboulet et al. (1994), Avalle et al., (1998)].

Another approach to identifying PILS analogs is provided by the use of combinatorial libraries. Methods for constructing and screening phage display and other combinatorial libraries are provided, for example, by [Kay et al., Phage Display of Peptides and Proteins (Academic Press 1996), U.S. 5,783,384, U.S. 5,747,334, and U.S. 5,723,323.

One illustrative in vitro use of PILS and its analogs is the production of labeled peptides from a labeled protein substrate. Proteases can also be used in detergents and cleaning solutions. For example, serine proteases are used in solutions to clean and to disinfect contact lenses (see, for example, [U.S. 5,985,629]). Another use for a serine protease is in the formulation of vaccines (see, for example, [U.S. 5,885,814]). Those of skill in the art can devise other uses for molecules having PILS activity.

*Antibodies*

Any type of antibody known in the art can be generated to bind specifically to an epitope of PILS.

"Antibody" as used herein includes intact immunoglobulin molecules, as well as fragments thereof, such as Fab, $F(ab')_2$, and Fv, which are capable of binding an epitope of PILS. Typically, at least 6, 8, 10, or 12 contiguous amino acids are required to form an epitope. However, epitopes which involve non-contiguous amino acids may require more, e.g., at least 15, 25, or 50 amino acid. An antibody which specifically binds to an epitope of PILS can be used therapeutically, as well as in immunochemical assays, such as Western blots, ELISAs, radioimmunoassays, immunohistochemical assays, immunoprecipitations, or other immunochemical assays known in the art. Various immunoassays can be used to identify antibodies having the desired specificity. Numerous protocols for competitive binding or immunoradiometric assays are well known in the art. Such immunoassays typically involve the measurement of complex formation between an immunogen and an antibody which specifically binds to the PILS immunogen.

Typically, an antibody which specifically binds to PILS provides a detection signal at least 5-, 10-, or 20-fold higher than a detection signal provided with other proteins when used in an immunochemical assay. Preferably, antibodies which specifically bind to PILS do not detect other proteins in immunochemical assays and can immunoprecipitate PILS from solution.

PILS can be used to immunize a mammal, such as a mouse, rat, rabbit, guinea pig, monkey, or human, to produce polyclonal antibodies. If desired, PILS can be conjugated to a carrier protein, such as bovine serum albumin, thyroglobulin, and keyhole limpet hemocyanin. Depending on the host species, various adjuvants can be used to increase the immunological response. Such adjuvants include, but are not limited to, Freund's adjuvant, mineral gels (e.g., aluminum hydroxide), and surface active substances (e.g., lysolecithin, pluronic polyols, polyanions, peptides, oil emulsions, keyhole limpet hemocyanin, and dinitrophenol). Among adjuvants used in humans, BCG (*bacilli Calmette-Guerin*) and *Corynebacterium parvum* are especially useful.

Monoclonal antibodies which specifically bind to PILS can be prepared using any technique which provides for the production of antibody m olecules by continuous cell lines in culture. These techniques include, but are not limited to, the hybridoma technique, the human B-cell hybridoma technique, and the EBV-hybridoma technique [Roberge, (1995)].

In addition, techniques developed for the production of "chimeric antibodies", the splicing of mouse antibody genes to human antibody genes to obtain a molecule with appropriate antigen specificity and biological activity, can be used. Monoclonal and other antibodies also can be "humanized" to prevent a patient from mounting an immune r esponse a gainst t he a ntibody w hen it is used therapeutically. Such antibodies may be sufficiently similar in sequence to human antibodies to be used directly in therapy or may require alteration of a few key residues. Sequence differences between rodent antibodies and human sequences can b e m inimized b y replacing residues which differ from those in the human sequences by site directed mutagenesis of individual residues or by grating of entire complementarity determining regions. Antibodies which specifically bind to PILS can contain antigen binding sites which are either partially or fully humanized, as disclosed in U.S. 5,565,332.

Alternatively, techniques described for the production of single chain antibodies can be adapted using methods known in the art to produce single chain antibodies which specifically bind to PILS. Antibodies with related specificity, but of distinct idiotypic composition, can be generated by chain shuffling from random combinatorial immunoglobin libraries. Single-chain antibodies also can be constructed using a DNA amplification method, such as PCR, using hybridoma cDNA as a template. Single-chain antibodies can be mono- or bispecific, and can be bivalent or tetravalent. Construction of tetravalent, bispecific single-chain antibodies is taught. A nucleotide sequence encoding a single-chain antibody can be constructed using manual or automated nucleotide synthesis, cloned into an expression construct using standard recombinant DNA methods, and introduced into a cell to express the coding sequence, as described below. Alternatively, single-chain antibodies can be produced directly using, for example, filamentous phage technology.

Antibodies which specifically bind to PILS also can be produced by inducing *in vivo* production in the lymphocyte population or by screening immunoglobulin libraries or panels of highly specific binding reagents. Other types of antibodies can be constructed and used therapeutically in methods related to the invention. For example, chimeric antibodies can be constructed as disclosed in WO 93/03151. Binding proteins which are derived from immunoglobulins and which are multivalent and multispecific, such as the "diabodies" described in WO 94/13804, also can be prepared.

Antibodies used in the invention can be purified by methods well known in the art. For example, antibodies can be affinity purified by passage over a column to which P ILS is bound. The bound antibodies c an then bee luted from the column using a buffer with a high salt concentration.

*Antisense Oligonucleotides*

Antisense oligonucleotides are nucleotide sequences which are complementary to a specific DNA or RNA sequence. Once introduced into a cell, the complementary nucleotides combine with natural sequences produced by the cell to form complexes and block either transcription or translation. Preferably, an antisense oligonucleotide is at least 11 nucleotides in length, but can be at least 12, 15, 20, 25, 30, 35, 40, 45, or 50 or more nucleotides long. Longer sequences also can be used. Antisense oligonucleotide molecules can be provided in a DNA construct and introduced into a cell as described above to decrease the level of PILS gene products in the cell.

Antisense oligonucleotides can be deoxyribonucleotides, ribonucleotides, or a combination of both. Oligonucleotides can be synthesized manually or by an automated synthesizer, by covalently linking the 5' end of one nucleotide with the 3' end of another nucleotide with non-phosphodiester internucleotide linkages such alkylphosphonates, phosphorothioates, phosphorodithioates, alkylphosphonothioates, alkylphosphonates, phosphoramidates, phosphate esters, carbamates, acetamidate, carboxymethyl esters, carbonates, and phosphate triesters.

Modifications of PILS gene expression can be obtained by designing antisense oligonucleotides which will form duplexes to the control, 5', or regulatory regions of the PILS gene. Oligonucleotides derived from the transcription initiation site, *e.g.,* between positions -10 and +10 from the start site, are preferred. Similarly, inhibition can be achieved using "triple helix" base-pairing methodology. Triple helix pairing is useful because it causes inhibition of the ability of the double helix to open sufficiently for the binding of polymerases, transcription factors, or chaperons. Therapeutic advances using triplex DNA have been described in the literature [Nicholls, (1993)]. An antisense oligonucleotide also can be designed to block translation of mRNA by preventing the transcript from binding to ribosomes.

Precise complementarity is not required for successful complex formation between an antisense oligonucleotide and the complementary sequence of a PILS polynucleotide. Antisense oligonucleotides which comprise, for example, 2, 3, 4, or 5 or more stretches of contiguous nucleotides which are precisely complementary to a PILS polynucleotide, each separated by a stretch of contiguous nucleotides which are not complementary to adjacent PILS nucleotides, can provide sufficient targeting specificity for PILS mRNA. Preferably, each stretch of complementary contiguous nucleotides is at least 4, 5, 6, 7, or 8 or more nucleotides in length. Non-complementary intervening sequences are preferably 1, 2, 3, or 4 nucleotides in length. One skilled in the art can easily use the calculated melting point of an antisense-sense pair to determine the degree of mismatching which will be tolerated between a particular antisense oligonucleotide and a particular PILS polynucleotide sequence. Antisense oligonucleotides can be modified without affecting their ability to hybridize to a PILS polynucleotide. These modifications can be internal or at one or both ends of the antisense molecule. For example, internucleoside phosphate linkages can be modified by adding cholesteryl or diamine moieties with varying numbers of carbon residues between the amino groups and terminal ribose. Modified bases and/or sugars, such as arabinose instead of ribose, or a 3', 5'-substituted oligonucleotide in which the 3' hydroxyl group or the 5' phosphate group are substituted, also can be employed in a modified antisense oligonucleotide. These modified oligonucleotides can be prepared by methods well known in the art.

*Ribozymes*

Ribozymes are RNA molecules with catalytic activity [Uhlmann, (1987)]. Ribozymes can be used to inhibit gene function by cleaving an RNA sequence, as is known in the art. The mechanism of ribozyme action involves sequence-specific hybridization of the ribozyme molecule to complementary target RNA, followed by endonucleolytic cleavage. Examples include engineered hammerhead motif ribozyme molecules that can specifically and efficiently catalyze endonucleolytic cleavage of specific nucleotide sequences. The coding sequence of a PILS polynucleotide can be used to generate ribozymes which will specifically bind to mRNA transcribed from a PILS polynucleotide. Methods of designing and constructing ribozymes which can cleave other RNA molecules in trans in a highly sequence specific manner have been developed and described in the art. For example, the cleavage activity of ribozymes can b e t argeted t o specific RNAs b y e ngineering a discrete "hybridization" region into the ribozyme. The hybridization region contains a sequence complementary to the target RNA and thus specifically hybridizes with the target RNA.

Specific ribozyme cleavage sites within a PILS RNA target can be identified by scanning the target molecule for ribozyme cleavage sites which include the following sequences: GUA, GUU, and GUC. Once identified, short RNA sequences of between 15 and 20 ribonucleotides corresponding to the region of the target RNA containing the cleavage site can be evaluated for secondary structural features which may render the target inoperable. Suitability of candidate PILS RNA targets also can be evaluated by testing accessibility to hybridization with complementary oligonucleotides using ribonuclease protection assays. The nucleotide sequences shown in SEQ ID NO: 1 and its complement provide sources of suitable hybridization region sequences. Longer complementary sequences can be used to increase the affinity of the hybridization sequence for the target. The hybridizing and cleavage regions of the ribozyme can be integrally related such that upon hybridizing to the target RNA through the complementary regions, the catalytic region of the ribozyme can cleave the target.

Ribozymes can be introduced into cells as part of a DNA construct. Mechanical methods, such as microinjection, liposome-mediated transfection, electroporation, or calcium phosphate precipitation, can be used to introduce a ribozyme-containing DNA construct into cells in which it is desired to decrease PILS expression. Alternatively, if it is desired that the cells stably retain the DNA construct, the construct can be supplied on a plasmid and maintained as a separate element or integrated into the genome of the cells, as is known in the art. A ribozyme-encoding DNA construct can include transcriptional regulatory elements, such as a promoter element, an enhancer or UAS element, and a transcriptional terminator s ignal, for controlling transcription of ribozymes in the cells (U.S. 5,641,673). Ribozymes also can be engineered to provide an additional level of regulation, so that destruction of mRNA occurs only when both a ribozyme and a target gene are induced in the cells.

**Screening / Screening Assays**

*Regulators*

Regulators as used herein, refer to compounds that affect the activity of PILS in vivo and/or in vitro. Regulators can be agonists and antagonists of PILS polypeptide and can be compounds that exert their effect on the PILS activity via the enzymatic activity, expression, post-translational modifications or by other means. Agonists of PILS are molecules which, when bound to PILS, increase or prolong the activity of PILS. Agonists of PILS include proteins, nucleic acids, carbohydrates, small molecules, or any other molecule which activate PILS. Antagonists of PILS are molecules which, when bound to PILS, decrease the amount or the duration of the activity of PILS. Antagonists include proteins, nucleic acids, carbohydrates, antibodies, small molecules, or any other molecule which decrease the activity of PILS.

The term "modulate", as it appears herein, refers to a change in the activity of PILS polypeptide. For example, modulation may cause an increase or a decrease in enzymatic activity, binding characteristics, or any other biological, functional, or immunological properties of PLLS.

As used herein, the terms "specific binding" or "specifically binding" refer to that interaction between a protein or peptide and an agonist, an antibody, or an antagonist. The interaction is dependent upon the presence of a particular structure of the protein recognized by the binding molecule (i.e., the antigenic determinant or epitope). For example, if an antibody is specific for epitope "A" the presence of a polypeptide containing the epitope A, or the presence of free unlabeled A, in a reaction containing free labeled A and the antibody will reduce the amount of labeled A that binds to the antibody.

The invention provides methods (also referred to herein as "screening assays") for identifying compounds which can be used for the treatment of diseases related to PILS. The methods entail the identification of candidate or test compounds or agents (e.g., peptides, peptidomimetics, small molecules or other molecules) which bind to PILS and/or have a stimulatory or inhibitory effect on the biological activity of PILS or its expression and then determining which of these compounds have an effect on symptoms or diseases related to PILS in an *in vivo* assay.

Candidate or test compounds or agents which bind to PILS and/or have a stimulatory or inhibitory effect on the activity or the expression of PILS are identified either in assays that employ cells which express P ILS (cell-based a ssays) or in assays with isolated PILS (cell-free assays). The various assays can employ a variety of variants of PILS (e.g., full-length PILS, a biologically active fragment of PILS, or a fusion protein w hich i ncludes all or a portion of PILS). Moreover, PILS can be derived from any suitable mammalian species (e.g., human PILS, rat PILS or murine PILS). The assay can be a binding assay entailing direct or indirect measurement of the binding of a test compound or a known PILS ligand to PILS. The assay can also be an activity assay entailing direct or indirect measurement of the activity of PILS. The assay can also be an expression assay entailing direct or indirect measurement of the expression of PILS mRNA or PILS protein. The various screening assays are combined with an in *vivo* assay entailing measuring the effect of the test compound on the symptoms of diseases related to PILS.

The present invention includes biochemical, cell free assays that allow the identification of i nhibitors a nd a gonists o f p roteases s uitable as lead structures for pharmacological drug development. Such assays involve contacting a form of PILS (e.g., full-length PILS, a biologically active fragment of PILS, or a fusion protein comprising all or a portion of PILS) with a test compound and determining the ability of the test compound to act as an antagonist (preferably) or an agonist of the enzymatic activity of PILS.

The activity of PILS molecules of the present invention can be measured using a variety of assays that measure PILS activity. For example, PILS enzyme activity can be assessed by a standard in vitro serine/metallo/... protease assay (see, for example, [U.S. 5,057,414]). Those of skill in the art are aware of a variety of substrates suitable for in vitro assays, such as SucAla-Ala-Pro-Phe-pNA, fluorescein mono-p-guanidinobenzoate hydrochloride, benzyloxycarbonyl-L-Arginyl-S-benzylester, Nalpha-Benzoyl-L-arginine ethyl ester hydrochloride, and the like. In addition, protease assay kits available from commercial sources, such as Calbiochem™ (San Diego, Calif.). For general references, see Barrett (Ed.), Methods in Enzymology, Proteolytic Enzymes: Serine and Cysteine Peptidases (Academic Press Inc. 1994), and Barrett et al., (Eds.), Handbook of Proteolytic Enzymes (Academic Press Inc. 1998).

Solution in vitro assays can be used to identify a PILS substrate or inhibitor. Solid phase systems can also be used to identify a substrate or inhibitor of a PILS polypeptide. For example, a PILS polypeptide or PILS fusion protein can be immobilized onto the surface of a receptor chip of a commercially available biosensor instrument (BIACORE, Biacore A B; U ppsala, S weden). T he u se of t his instrument is disclosed, for example, by [Karlsson, (1991), and Cunningham and Wells, (1993)].

In brief, a PILS polypeptide or fusion protein is covalently attached, using amine or sulfhydryl chemistry, to dextran fibers that are attached to gold film within a flow cell. A test sample is then passed through the cell. If a PILS substrate or inhibitor is present in the sample, it will bind to the immobilized polypeptide or fusion protein, causing a change in the refractive index of the medium, which is detected as a change in surface plasmon resonance of the gold film. This system allows the determination on- and off-rates, from which binding affinity can be calculated, and assessment of the stoichiometry of binding, as well as the kinetic effects of PILS mutation. This system can also be used to examine antibody-antigen interactions, and the interactions of other complement/anti-complement pairs.

In one embodiment, the invention provides assays for screening candidate or test compounds which bind to or modulate the activity of PILS. Such assays can employ full-length PILS, a biologically active fragment of PILS, or a fusion protein which includes all or a portion of PILS. As described in greater detail below, the test compound can be obtained by any suitable means, e.g., from conventional compound libraries.

Determining the ability of the test compound to modulate the activity of PILS can be accomplished, for example, by determining the ability of PILS to bind to or interact with a target molecule. The target molecule can be a molecule with which PILS binds or interacts with in nature. The target molecule can be a component of a signal transduction pathway which facilitates transduction of an extracellular signal. The target PILS molecule can be, for example, a second intracellular protein which has catalytic activity or a protein which facilitates the association of downstream signaling molecules with PILS.

Determining the ability of PILS to bind to or interact with a target molecule can be accomplished by one of the methods described above for determining direct binding. In one embodiment, determining the ability of a polypeptide of the invention to bind to or interact with a target molecule can be accomplished by determining the activity of the target molecule. For example, the activity of the target molecule can be determined by detecting induction of a cellular second messenger of the target (e.g., intracellular $Ca^{2+}$, diacylglycerol, $IP_3$, etc.), detecting catalytic/enzymatic activity of the target on an appropriate substrate, detecting the induction of a reporter gene (e.g., a regulatory element that is responsive to a polypeptide of the invention operably linked to a nucleic acid encoding a detectable marker, e.g., luciferase), or detecting a cellular response.

In various embodiments of the above assay methods of the present invention, it may be desirable to immobilize PILS (or a PILS target molecule) to facilitate separation of complexed from uncomplexed forms of one or both of the proteins, as well as to accommodate automation of the assay. Binding of a test compound to PILS, or interaction of PILS with a target molecule in the presence and absence of a candidate compound, can be accomplished in any vessel suitable for containing the reactants. Examples of such vessels include microtitre plates, test tubes, and micro-centrifuge tubes. In one embodiment, a fusion protein can be provided which adds a domain that allows one or both of the proteins to be bound to a matrix. For example, glutathione-S-transferase (GST) fusion proteins or glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed target protein or PILS, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of PILS can be determined using standard techniques.

Other techniques for immobilizing proteins on matrices can also be used in the screening assays of the invention. For example, either PILS or its target molecule can be immobilized utilizing conjugation of biotin and streptavidin. Biotinylated polypeptide used in the invention or target molecules can be prepared from biotin-NHS (N-hydroxy-succinimide) using techniques well known in the art (e.g., biotinylation kit, Pierce Chemicals; Rockford, III), and immobilized in the wells of streptavidin-coated plates (Pierce Chemical). Alternatively, antibodies reactive with PILS or target molecules but which do not interfere with binding of the polypeptide used in the invention to its target molecule can be derivatized to the wells of the plate, and unbound target or polypeptide used in the invention trapped in the wells by antibody conjugation. Methods for detecting such complexes, in addition to those described above for the GST-immobilized complexes, include immunodetection of complexes using antibodies reactive with PILS or target molecule, as well as enzyme-linked assays which rely on detecting an enzymatic activity associated with PILS or target molecule.

Another technique for drug screening which may be used provides for high throughput screening of compounds having suitable binding affinity to the protein of interest as described in published PCT application WO84/03564. In this method, large numbers of different small test compounds are synthesized on a solid substrate, such as plastic pins or some other surface. The test compounds are reacted with PILS, or fragments thereof, and washed. Bound PILS is then detected by methods well known in the art. Purified PILS can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding PILS specifically compete with a testcompound for binding PILS. In this manner, antibodies can be used to detect the presence of any peptide which shares one or more antigenic determinants with PILS.

The screening assay can also involve monitoring the expression of PILS. For example, regulators of expression of PILS can be identified in a method in which a cell is contacted with a candidate compound and the expression of PILS protein or mRNA in the cell is determined. The level of expression of PILS protein or mRNA the presence of the candidate compound is compared to the level of expression of PILS protein or mRNA in the absence of the candidate compound. The candidate compound can then be identified as a regulator of expression of PILS based on this comparison. For example, when expression of PILS protein or mRNA protein is greater (statistically significantly greater) in the presence of the candidate compound than in its absence, the candidate compound is identified as a stimulator of PILS protein or mRNA expression. Alternatively, when expression of PILS protein or mRNA is less (statistically significantly less) in the presence of the candidate compound than in its absence, the candidate compound is identified as an inhibitor of PILS protein or mRNA expression. The level of PILS protein or mRNA expression in the cells can be determined by methods described below.

*Binding Assays*

For binding assays, the test compound is preferably a small molecule which binds to and occupies the active site of PILS polypeptide, thereby making the ligand binding site inaccessible to substrate such that normal biological activity is prevented. Examples of such small molecules include, but are not limited to, small peptides or peptide-like molecules. Potential ligands which bind to a polypeptide of the invention include, but are not limited to, the natural ligands of known PILS proteases and analogues or derivatives thereof.

In binding assays, either the test compound or the PILS polypeptide can comprise a detectable label, such as a fluorescent, radioisotopic, chemiluminescent, or enzymatic label, such as horseradish peroxidase, alkaline phosphatase, or luciferase. Detection of a test compound which is bound to PILS polypeptide can then be accomplished, for example, by direct counting of radioemmission, by scintillation counting, or by determining conversion of an appropriate substrate to a detectable product. Alternatively, binding of a test compound to a PILS polypeptide can be determined without labeling either of the interactants. For example, a microphysiometer can be used to detect binding of a test compound with a PILS polypeptide. A microphysiometer (e.g., Cytosensor™) is an analytical instrument that measures the rate at which a cell acidifies its environment using a light-addressable potentiometric sensor (LAPS). Changes in this acidification rate can be used as an indicator of the interaction between a test compound and PILS [Haseloff, (1988)].

Determining the ability of a test compound to bind to PILS also can be accomplished using a technology such as real-time Bimolecular Interaction Analysis (BIA) [McConnell, (1992); Sjolander, (1991)]. BIA is a technology for studying bio-specific interactions in real time, without labeling any of the interactants (e.g., BIAcore™). Changes in the optical phenomenon surface plasmon resonance (SPR) can be used as an indication of real-time reactions between biological molecules.

In yet another aspect of the invention, a PILS-like polypeptide can be used as a "bait protein" in a two-hybrid assay or three-hybrid assay [Szabo, (1995); U.S. 5,283,317), to identify other proteins which bind to or interact with PILS and modulate its activity.

The two-hybrid system is based on the modular nature of most transcription factors, which consist of separable DNA-binding and activation domains. Briefly, the assay utilizes two different DNA constructs. For example, in one construct, polynucleotide encoding PILS can be fused to a polynucleotide encoding the DNA binding domain of a known transcription factor (e.g., GAL-4). In the other construct a DNA sequence that encodes an unidentified protein ("prey" or "sample") can be fused to a polynucleotide that codes for the activation domain of the known transcription factor. If the "bait" and the "prey" proteins are able to interact *in vivo* to form an protein-dependent complex, the DNA-binding and activation domains of the transcription factor are brought into close proximity. This proximity allows transcription of a reporter gene (e.g., LacZ), which is operably linked to a transcriptional regulatory site responsive to the transcription factor. Expression of the reporter gene can be detected, and cell colonies containing the functional transcription factor can be isolated and used to obtain the DNA sequence encoding the protein which interacts with PILS.

It may be desirable to immobilize either the PILS (or polynucleotide) or the test compound to facilitate separation of the bound form from unbound forms of one or both of the interactants, as well as to accommodate automation of the assay. Thus, either the PILS-like polypeptide (or polynucleotide) or the test compound can be bound to a solid support. Suitable solid supports include, but are not limited to, glass or plastic slides, tissue culture plates, microtiter wells, tubes, silicon chips, or particles such as beads (including, but not limited to, latex, polystyrene, or glass beads). Any method known in the art can be used to attach PILS-like polypeptide (or polynucleotide) or test compound to a solid support, including use of covalent and non-covalent linkages, passive absorption, or pairs of binding moieties attached respectively to the p olypeptide (or polynucleotide) or test compound and the solid support. Test compounds are preferably bound to the solid support in an array, so that the location of individual test compounds can be tracked. Binding of a test compound to PILS (or a polynucleotide encoding for PILS) can be accomplished in any vessel suitable for containing the reactants. Examples of such vessels include microtiter plates, test tubes, and microcentrifuge tubes.

In one embodiment, PILS is a fusion protein comprising a domain that allows binding of PILS t o a solid s upport. F or e xample, g lutathione-S-transferase fusion proteins can be adsorbed onto glutathione sepharose beads (Sigma Chemical, St. Louis, Mo.) or glutathione derivatized microtiter plates, which are then combined with the test compound or the test compound and the non-adsorbed PILS; the mixture is then incubated under conditions conducive to complex formation (e.g., at physiological conditions for salt and pH). Following incubation, the beads or microtiter plate wells are washed to remove any unbound components. Binding of the interactants can be determined either directly or indirectly, as described above.

Alternatively, the complexes can be dissociated from the solid support before binding is determined.

Other techniques for immobilizing proteins or polynucleotides on a solid support also can be used in the screening assays of the invention. For example, either PILS (or a polynucleotide encoding PILS) or a test compound can be immobilized utilizing conjugation of biotin and streptavidin. Biotinylated PILS (or a polynucleotide encoding biotinylated PILS) or test compounds can be prepared from biotin-NHS (N-hydroxysuccinimide) using techniques well known in the art (e.g., biotinylation kit, Pierce Chemicals, Rockford, Ill.) and immobilized in the wells of streptavidin-coated plates (Pierce Chemical). Alternatively, antibodies which specifically bind to PILS, polynucleotide, or a test compound, but which do not interfere with a desired binding site, such as the active site of PILS, can be derivatized to the wells of the plate. Unbound target or protein can be trapped in the wells by antibody conjugation.

Methods for detecting such complexes, in addition to those described above for the GST-immobilized complexes, include immunodetection of complexes using antibodies which specifically bind to PILS polypeptide or test compound, enzyme-linked assays which rely on detecting an activity of PILS polypeptide, and SDS gel electrophoresis under non-reducing conditions.

Screening for test compounds which bind to a PILS polypeptide or polynucleotide also can be carried out in an intact cell. Any c ell which c omprises a P ILS p olypeptide or polynucleotide can be used in a cell-based assay system. A PILS polynucleotide can be naturally occurring in the cell or can be introduced using techniques such as those described above. Binding of the test compound to PILS or a polynucleotide encoding PILS is determined as described above.

*Functional Assays*

Test compounds can be tested for the ability to increase or decrease PILS activity of a PILS polypeptide. The PILS activity can be measured, for example, using methods described in the specific examples, below. PILS activity can be measured after contacting either a purified PILS or an intact cell with a test compound. A test compound which decreases PILS activity by at least about 10, preferably about 50, more preferably about 75, 90, or 100% is identified as a potential agent for decreasing PILS activity. A test compound which increases PILS activity by at least about 10, preferably about 50, more preferably about 75, 90, or 100% is identified as a potential agent for increasing PILS activity.

*Gene Expression*

In another embodiment, test compounds which increase or decrease PILS gene expression are identified. As used herein, the term "correlates with expression of a polynucleotide" indicates that the detection of the presence of nucleic acids, the same or related to a nucleic acid sequence encoding PILS, by northern analysis or realtime PCR is indicative of the presence of nucleic acids encoding PILS in a sample, and thereby correlates with expression of the transcript from the polynucleotide encoding PILS. The term "microarray", as used herein, refers to an array of distinct polynucleotides or oligonucleotides arrayed on a substrate, such as paper, nylon or any other type of membrane, filter, chip, glass slide, or any other suitable solid support. A PILS polynucleotide is contacted with a test compound, and the expression of an RNA or polypeptide product of PILS polynucleotide is determined. The level of expression of appropriate mRNA or polypeptide in the presence of the test compound is compared to the level of expression of mRNA or polypeptide in the absence of the test compound. The test compound can then be identified as a regulator of expression based on this comparison. For example, when expression of mRNA or polypeptide is greater in the presence of the test compound than in its absence, the test compound is identified as a stimulator or enhancer of the mRNA or polypeptide expression. Alternatively, when expression of the mRNA or polypeptide is less in the presence of the test compound than in its absence, the test compound is identified as an inhibitor of the mRNA or polypeptide expression.

The level of PILS mRNA or polypeptide expression in the cells can be determined by methods well known in the art for detecting mRNA or polypeptide. Either qualitative or quantitative methods can be used. The presence of polypeptide products of PILS polynucleotide can be determined, for example, using a variety of techniques known in the art, including immunochemical methods such as radioimmunoassay, Western blotting, and immunohistochemistry. Alternatively, polypeptide synthesis can be determined *in vivo*, in a cell culture, or in an *in vitro* translation system by detecting incorporation of labelled amino acids into PILS.

Such screening can be carried out either in a cell-free assay system or in an intact cell. Any cell which expresses PILS polynucleotide can be used in a cell-based assay system. The PILS polynucleotide can be naturally occurring in the cell or can be introduced using techniques such as those described above. Either a primary culture or an established cell line can be used.

*Test Compounds*

Suitable test compounds for use in the screening assays of the invention can be obtained from any suitable source, e.g., conventional compound libraries. The test compounds can also be obtained using any of the numerous approaches in combinatorial library methods known in the art, including: biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds [Lam, (1997)]. Examples of methods for the synthesis of molecular libraries can be found in the art. Libraries of compounds may be presented in solution or on beads, bacteria, spores, plasmids or phage.

## Therapeutic Indications and Methods

It was found by the present applicant that PILS is expressed in various human tissues.

*Cardiovascular Disorders*

Heart failure is defined as a pathophysiological state in which an abnormality of cardiac function is responsible for the failure of the heart to pump blood at a rate commensurate with the requirement of the metabolizing tissue. It includes all forms of pumping failures such as high-output and low-output, acute and chronic, right-sided or left-sided, systolic or diastolic, independent of the underlying cause.

Myocardial infarction (MI) is generally caused by an abrupt decrease in coronary blood flow that follows a thrombotic occlusion of a coronary artery previously narrowed by arteriosclerosis. MI prophylaxis (primary and secondary prevention) is included as well as the acute treatment of MI and the prevention of complications.

Ischemic diseases are conditions in which the coronary flow is restricted resulting in a perfusion which is inadequate to meet the myocardial requirement for oxygen. This group of diseases includes stable angina, unstable angina and asymptomatic ischemia.

Arrhythmias include all forms of atrial and ventricular tachyarrhythmias, atrial tachycardia, atrial flutter, atrial fibrillation, atrio-ventricular reentrant tachycardia, preexitation syndrome, ventricular tachycardia, ventricular flutter, ventricular fibrillation, as well as bradycardic forms of arrhythmias.

Atherosclerosis is a cardiovascular disease in which the vessel wall is remodeled, compromising the lumen of the vessel. The atherosclerotic remodeling process involves accumulation of cells, both smooth muscle cells and monocyte/macrophage inflammatory cells, in the intima of the vessel wall. These cells take up lipid, likely from the circulation, to form a mature atherosclerotic lesion. Although the formation of these lesions is a chronic process, occurring over decades of an adult human life, the majority of the morbidity associated with atherosclerosis occurs when a lesion ruptures, releasing thrombogenic debris that rapidly occludes the artery. When such an acute event occurs in the coronary artery, myocardial infarction can ensue, and in the worst case, can result in death.

The formation of the atherosclerotic lesion can be considered to occur in five overlapping stages such as migration, lipid accumulation, recruitment of inflammatory cells, proliferation of vascular smooth muscle cells, and extracellular matrix deposition. Each of these processes can be shown to occur in man and in animal models of atherosclerosis, but the relative contribution of each to the pathology and clinical significance of the lesion is unclear.

Thus, a need exists for therapeutic methods and agents to treat cardiovascular pathologies, such as atherosclerosis and other conditions related to coronary artery disease.

Cardiovascular diseases include but are not limited to disorders of the heart and the vascular system like congestive heart failure, myocardial infarction, ischemic diseases of the heart, all kinds of atrial and ventricular arrhythmias, and atherosclerosis.

The risk to develop atherosclerosis and coronary artery or carotid artery disease (and thus the risk of having a heart attack or stroke) increases with the total cholesterol level increasing. Nevertheless, extremely low cholesterol levels may not be healthy. Examples of disorders of lipid metabolism are hyperlipidemia (abnormally high levels of fats (cholesterol, triglycerides, or both) in the blood, may be caused by family history of hyperlipidemia), obesity, a high-fat diet, lack of exercise, moderate to high alcohol consumption, cigarette smoking, poorly controlled diabetes, and an underactive thyroid gland), hereditary hyperlipidemias (type I hyperlipoproteinemia (familial hyperchylomicronemia), type II hyperlipoproteinemia (familial hypercholesterolemia), type III hyperlipoproteinemia, type IV hyperlipoproteinemia, or type V hyperlipoproteinemia), hypolipoproteinemia, lipidoses (caused by abnormalities in the enzymes that metabolize fats), Gaucher's disease, Niemann-Pick disease, Fabry's disease, Wolman's disease, cerebrotendinous xanthomatosis, sitosterolemia, Refsum's disease, or Tay-Sachs disease.

The human PILS is highly expressed in the following cardiovascular related tissues: heart, heart atrium (left), aortic endothel cells, coronary artery endothel cells. Expression in the above mentioned tissues demonstrates that the human PILS or mRNA can be utilized to diagnose of heart failure, myocardial infarction, ischemic diseases of the heart, arrhythmias, atherosclersois in the coronary artery or aorta. Additionally the activity of the human PILS can be modulated to treat heart failure, myocardial infarction, ischemic diseases of the heart, arrhythmias, atherosclersois in the coronary artery or aorta.

*Applications*

The present invention provides for both prophylactic and therapeutic methods for heart failure, myocardial infarction, ischemic diseases of the heart, arrhythmias, atherosclersois in the coronary artery or aorta.

The regulatory method of the invention involves contacting a cell with an agent that modulates one or more of the activities of PILS. An agent that modulates activity can be an agent as described herein, such as a nucleic acid or a protein, a naturally-occurring cognate ligand of the polypeptide, a peptide, a peptidomimetic, or any small molecule. In one embodiment, the agent stimulates one or more of the biological activities of PILS. Examples of such stimulatory agents include the active PILS and nucleic acid molecules encoding a portion of PILS. In another embodiment, the agent inhibits one or more of the biological activities of PILS. Examples of such inhibitory agents include antisense nucleic acid molecules and antibodies. These regulatory methods can be performed in vitro (e.g., by culturing the cell with the agent) or, alternatively, in vivo (e.g, by administering the agent to a subject). As such, the present invention provides methods of treating an individual afflicted with a disease or disorder characterized by unwanted expression or activity of PILS or a protein in the PILS signaling pathway. In one embodiment, the method involves administering an agent like any agent identified or being identifiable by a screening assay as described herein, or combination of such agents that modulate say upregulate or downregulate the expression or activity of PILS or of any protein in the PILS signaling pathway. In another embodiment, the method involves administering a regulator of PILS as therapy to compensate for reduced or undesirably low expression or activity of PILS or a protein in the PILS signaling pathway.

Stimulation of activity or expression of PILS is desirable in situations in which enzymatic activity or expression is abnormally low and in which increased activity is likely to have a beneficial effect. Conversely, inhibition of enzymatic activity or expression of PILS is desirable in situations in which activity or expression of PILS is abnormally high and in which decreasing its activity is likely to have a beneficial effect.

This invention is further illustrated by the following examples which should not be construed as limiting. The contents of all references, patents and published patent applications cited throughout this application are hereby incorporated by reference.

**Pharmaceutical Compositions**

This disclosure further pertains to novel agents identified by the above-described screening assays and uses thereof for treatments as described herein.

The nucleic acid molecules, polypeptides, and antibodies (also referred to herein as "active compounds") used in the invention can be incorporated into pharmaceutical compositions suitable for administration. Such compositions typically comprise the nucleic acid molecule, protein, or antibody and a pharmaceutically acceptable carrier.

As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes use of a PILS polynucleotide or polypeptide, or, an antibody, a ribozyme or an antisense oligonucleotide which regulate the activity of a PILS polypeptide for the preparation of a pharmaceutical composition useful in the treatment of heart failure, myocardial infarction, ischemic diseases of the heart, arrhythmias, atherosclersois in the coronary artery or aorta as well as methods for preparing such compositions by combining one or more such regulators and a pharmaceutically acceptable carrier.

An inhibitor of PILS may be produced using methods which are generally known in the art. In particular, purified PILS may be used to produce antibodies or to screen libraries of pharmaceutical agents to identify those which specifically bind PILS. Antibodies to PILS may also be generated using methods that are well known in the art. Such antibodies may include, but are not limited to, polyclonal, monoclonal, chimeric, single chain antibodies, Fab fragments, and fragments produced by a Fab expression library. Neutralizing antibodies like those which inhibit dimer formation are especially preferred for therapeutic use.

In another embodiment of the invention, the polynucleotides encoding PILS, or any fragment or complement thereof, may be used for therapeutic purposes. In one aspect, the complement of the polynucleotide encoding PILS may be used in situations in which it would be desirable to block the transcription of the mRNA. In particular, cells may be transformed with sequences complementary to polynucleotides encoding PILS. Thus, complementary molecules or fragments may be used to modulate PILS activity, or to achieve regulation of gene function. Such technology is now well known in the art, and sense or antisense oligonucleotides or larger fragments can be designed from various locations along the coding or control regions of sequences encoding PILS.

Expression vectors derived from retroviruses, adenoviruses, or herpes or vaccinia viruses, or from various bacterial plasmids, may be used for delivery of nucleotide sequences to the targeted organ, tissue, or cell population. Methods which are well known to those skilled in the art can be used to construct vectors which will express nucleic acid sequence complementary to the polynucleotides of the gene encoding PILS. These techniques are described, for example, in [Scott and Smith (1990)].

Any of the therapeutic methods described above may be applied to any subject in need of such therapy, including, for example, mammals such as dogs, cats, cows, horses, rabbits, monkeys, and most preferably, humans.

Disclosed is the administration of a pharmaceutical composition containing PILS in conjunction with a pharmaceutically acceptable carrier, for any of the therapeutic effects discussed above. Such pharmaceutical compositions may consist of PILS, antibodies to PILS, and mimetics, agonists, antagonists, or inhibitors of PILS. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs or hormones.

A pharmaceutical composition of the invention is formulated to be compatible with its intended route of administration. Examples of routes of administration include parenteral, e.g., intravenous, intradermal, subcutaneous, oral (e.g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions. For intravenous administration, suitable carriers include physiological saline, bacteriostatic water, Cremophor EM™ (BASF, Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, a pharmaceutically acceptable polyol like glycerol, propylene glycol, liquid polyetheylene glycol, and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin. Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed.

Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. 4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals.

Polynucleotide sequences encoding PILS may be used for the diagnosis of atherosclerosis in coronary artery or aorta associated with expression of PILS. The polynucleotide sequences encoding PILS may be used in Southern, Northern, or dot-blot analysis, or other membrane-based technologies; in PCR technologies; in dipstick, pin, and ELISA assays; and in microarrays utilizing fluids or tissues from patient biopsies to detect altered PILS expression. Such qualitative or quantitative methods are well known in the art.

In a particular aspect, the nucleotide sequences encoding PILS may be useful in assays that detect the presence of associated disorders, particularly those mentioned above. The nucleotide sequences encoding PILS may be labelled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered from that of a comparable control sample, the nucleotide sequences have hybridized with nucleotide sequences in the sample, and the presence of altered levels of nucleotide sequences encoding PILS in the sample indicates the presence of the associated disorder. Such assays may also be used to evaluate the efficacy of a particular therapeutic treatment regimen in animal studies, in clinical trials, or in monitoring the treatment of an individual patient.

In order to provide a basis for the diagnosis of atherosclerosis in coronary artery or aorta associated with expression of PILS, a normal or standard profile for expression is established. This may be accomplished by combining body fluids or cell extracts taken from normal subjects, either animal or human, with a sequence, or a fragment thereof, encoding PILS, under conditions suitable for hybridization or amplification. Standard hybridization may be quantified by comparing the values obtained from normal subjects with values from an experiment in which a known amount of a substantially purified polynucleotide is used. Standard values obtained from normal samples may be compared with values obtained from samples from patients who are symptomatic for a disorder. Deviation from standard values is used to establish the presence of a disorder.

**Determination of a Therapeutically Effective Dose**

The determination of a therapeutically effective dose is well within the capability of those skilled in the art. A therapeutically effective dose refers to that amount of active ingredient which increases or decreases PILS activity relative to PILS activity which occurs in the absence of the therapeutically effective dose. For any compound, the therapeutically effective dose can be estimated initially either in cell culture assays or in animal models, usually mice, rabbits, dogs, or pigs. The animal model also can be used to determine the appropriate concentration range and route of administration. Such information c an t hen be used t o determine useful doses and routes for administration in humans.

Therapeutic efficacy and toxicity, e.g., $ED_{50}$ (the dose therapeutically effective in 50% of the population) and $LD_{50}$ (the dose lethal to 50% of the population), can be determined by standard pharmaceutical procedures in cell cultures or experimental animals. The dose ratio of toxic to therapeutic effects is the therapeutic index, and it can be expressed as the ratio, $LD_{50}/ED_{50}$. Pharmaceutical compositions which exhibit large therapeutic indices are preferred. The data obtained from cell culture assays and animal studies is used in formulating a range of dosage for human use.

The dosage contained in such compositions is preferably within a range of circulating concentrations that include the $ED_{50}$ with little or no toxicity. The dosage varies within this range depending upon the dosage form employed, sensitivity of the patient, and the route of administration. The exact dosage will be determined by the practitioner, in light of factors related to the subject that requires treatment. Dosage and administration are adjusted to provide sufficient levels of the active ingredient or to maintain the desired effect. Factors which can be taken into account include the severity of the disease state, general health of the subject, age, weight, and gender of the subject, diet, time and frequency of administration, drug combination(s), reaction sensitivities, and tolerance/response to therapy. Long-acting pharmaceutical compositions can be administered every 3 to 4 days, every week, or once every two weeks depending on the half-life and clearance rate of the particular formulation.

Normal dosage amounts can vary from 0.1 micrograms to 100,000 micrograms, up to a total dose of about 1 g, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc. If the reagent is a single-chain antibody, polynucleotides encoding the antibody can be constructed and introduced into a cell either *ex vivo* or *in vivo* using well-established techniques including, but not limited to, transferrin-polycation-mediated DNA transfer, transfection with naked or encapsulated nucleic acids, liposome-mediated cellular fusion, intracellular transportation of DNA-coated latex beads, protoplast fusion, viral infection, electroporation, "gene gun", and DEAE- or calcium phosphate-mediated transfection.

If the expression product is mRNA, the reagent is preferably an antisense oligonucleotide or a ribozyme. Polynucleotides which express antisense oligonucleotides or ribozymes can be introduced into cells by a variety of methods, as described above. Preferably, a reagent reduces expression of PILS gene or the activity of PILS by at least about 10, preferably about 50, more preferably about 75, 90, or 100% relative to the absence of the reagent. The effectiveness of the mechanism chosen to decrease the level of expression of PILS gene or the activity of PILS can be assessed using methods well known in the art, such as hybridization of nucleotide probes to PILS-specific mRNA, quantitative RT-PCR, immunologic detection of PILS, or measurement of PILS activity.

In any of the embodiments described above, any of the pharmaceutical compositions of- the invention can be administered in combination with other appropriate therapeutic agents. Selection of the appropriate agents for use in combination therapy can be made by one of ordinary skill in the art, according to conventional pharmaceutical principles. The combination of therapeutic agents can act synergistically to effect the treatment or prevention of the various disorders described above. Using this approach, one may be able to achieve therapeutic efficacy with lower dosages of each agent, thus reducing the potential for adverse side effects. Any of the therapeutic methods described above can be applied to any subject in need of such therapy, including, for example, mammals such as dogs, cats, cows, horses, rabbits, monkeys, and most preferably, humans.

Nucleic acid molecules used in the invention are those nucleic acid molecules which are contained in a group of nucleic acid molecules consisting of (i) nucleic acid molecules encoding a polypeptide comprising the amino acid sequence of SEQ ID NO: 2, (ii) nucleic acid molecules comprising the sequence of SEQ ID NO: 1, (iii) nucleic acid molecules having the sequence of SEQ ID NO: 1, (iv)nucleic acid molecules the complementary strand of which hybridizes under stringent conditions to a nucleic acid molecule of (i), (ii), or (iii); and (v) nucleic acid molecules the sequence of which differs from the sequence of a nucleic acid molecule of (iii) due to the degeneracy of the genetic code, wherein the polypeptide encoded by said nucleic acid molecule has PILS activity.

Polypeptides used in the invention are those polypeptides which are contained in a group of polypeptides consisting of (i) polypeptides having the sequence of SEQ ID NO: 2, (ii) polypeptides comprising the sequence of SEQ ID NO: 2, (iii) polypeptides encoded by nucleic acid molecules used in the invention and (iv) polypeptides which show at least 99%, 98%, 95%, 90%, or 80% homology with a polypeptide of (i), (ii), or (iii), wherein said purified polypeptide has PILS activity.

An object of the invention is a method of screening for therapeutic agents useful in the treatment of a disease comprised in a group of diseases consisting of heart failure, myocardial infarction, ischemic diseases of the heart, arrhythmias, atherosclersois in the coronary artery or aorta in a mammal comprising the steps of (i) contacting a test compound with a PILS polypeptide, (ii) detect binding of said test compound to said PILS polypeptide. E.g., compounds that bind to the PILS polypeptide are identified potential therapeutic agents for such a disease.

Another object of the invention is a method of screening for therapeutic agents useful in the treatment of a disease comprised in a group of diseases consisting of heart failure, myocardial infarction, ischemic diseases of the heart, arrhythmias, atherosclersois in the coronary artery or aorta in a mammal comprising the steps of (i) determining the activity of a PILS polypeptide at a certain concentration of a test compound or in the absence of said test compound, (ii) determining the activity of said polypeptide at a different concentration of said test compound. E.g., compounds that lead to a difference in the activity of the PILS polypeptide in (i) and (ii) are identified potential therapeutic agents for such a disease.

Another object of the invention is a method of screening for therapeutic agents useful in the treatment of a disease comprised in a group of diseases consisting of heart failure, myocardial infarction, ischemic diseases of the heart, arrhythmias, atherosclersois in the coronary artery or aorta in a mammal comprising the steps of (i) determining the activity of a PILS polypeptide at a certain concentration of a test compound, (ii) determining the activity of a PILS polypeptide at the presence of a compound known to be a regulator of a PILS polypeptide. E.g., compounds that show similar effects on the activity of the PILS polypeptide in (i) as compared to compounds used in (ii) a re identified potential therapeutic a gents for such a disease.

Other objects of the invention are methods of the above, wherein the step of contacting is in or at the surface of a cell.

Other objects of the invention are methods of the above, wherein the cell is in vitro.

Other objects of the invention are methods of the above, wherein the step of contacting is in a cell-free system.

Other objects of the invention are methods of the above, wherein the polypeptide is coupled to a detectable label.

Other objects of the invention are methods of the above, wherein the compound is coupled to a detectable label.

Other objects of the invention are methods of the above, wherein the test compound displaces a ligand which is first bound to the polypeptide.

Other objects of the invention are methods of the above, wherein the polypeptide is attached to a solid support.

Other objects of the invention are methods of the above, wherein the compound is attached to a solid support.

Another object of the invention is a method of screening for therapeutic agents in the treatment of a disease comprised in a group of diseases consisting of heart failure, myocardial infarction, ischemic diseases of the heart, arrhythmias, atherosclersois in the coronary artery or aorta in a mammal comprising the steps of (i) contacting a test compound with a PILLS polynucleotide, (ii) detect binding of said test compound to said PILS polynucleotide. Compounds that, e.g., bind to the PILS polynucleotide are potential therapeutic agents for the treatment of such diseases.

Another object of the invention is the method of the above, wherein the nucleic acid molecule is RNA.

Another object of the invention is a method of the above, wherein the contacting step is in or at the surface of a cell.

Another object of the invention is a method of the above, wherein the contacting step is in a cell-free system.

Another object of the invention is a method of the above, wherein the polynucleotide is coupled to a detectable label.

Another object of the invention is a method of the above, wherein the test compound is coupled to a detectable label.

Another object of the invention is a method of diagnosing atherosclerosis in coronary artery or aorta in a mammal comprising the steps of (i) determining the amount of a PILS polynucleotide in a sample taken from said mammal, (ii) determining the amount of PILS polynucleotide in healthy and/or diseased mammal. A disease is diagnosed, e.g., if there is a substantial similarity in the amount of PILS polynucleotide in said test mammal as compared to a diseased mammal.

Another object of the invention is a pharmaceutical composition for the treatment of a disease comprised in a group of diseases consisting of heart failure, myocardial infarction, ischemic diseases of the heart, arrhythmias, atherosclersois in the coronary artery or aorta in a mammal comprising a therapeutic agent which regulates the activity of a PILS polypeptide, wherein said therapeutic agent is (i) an antisense oligonucleotide, (ii) an antibody, or (iii) a ribozyme.

Another object of the invention is a pharmaceutical composition for the treatment of a disease comprised in a group of diseases consisting of heart failure, myocardial infarction, ischemic diseases of the heart, arrhythmias, atherosclersois in the coronary artery or aorta in a mammal comprising a PILS polynucleotide.

Another object of the invention is a pharmaceutical composition for the treatment of a disease comprised in a group of diseases consisting of heart failure, myocardial infarction, ischemic diseases of the heart, arrhythmias, atherosclersois in the coronary artery or aorta in a mammal comprising a PILS polypeptide.

The examples below are provided to illustrate the subject invention. These examples are provided by way of illustration and are not included for the purpose of limiting the invention.

## Examples

### Example 1: Search for homologous sequences in public sequence data bases

The degree of homology can readily be calculated by known methods. Preferred methods to determine homology are designed to give the largest match between the sequences tested. Methods to determine homology are codified in publicly available computer programs such as BestFit, BLASTP, BLASTN, and FASTA. The BLAST programs are publicly available from NCBI and other sources in the internet.

For PILS the following hits to known sequences were identified by using the BLAST algorithm [Altschul SF, Madden TL, Schaffer AA, Zhang J, Zhang Z, Miller W, Lipman DJ; Nucleic Acids Res 1997 Sep 1; 25(17): 3389-402] and the following set of parameters: matrix = BLOSUM62 and low complexity filter. The following databases were searched: NCBI (non-redundant database) and DERWENT patent database (Geneseq).

The following hits were found:

>gb|AAF20384.1|AF183569_1 aminopeptidase PILS [Homo sapiens] Length = 941 Score = 1904 bits (4932), Expect = 0.0 Identities = 941/941 (100%), Positives = 941/941 (100%)

>gb|AAF34664.1|AF222340_1 type 1 tumor necrosis factor receptor shedding aminopeptidase regulator [Homo sapiens] gb|AAH30775.1| type 1 tumor necrosis factor receptor shedding aminopeptidase regulator [Homo sapiens] Length = 941 Score = 1896 bits (4912), Expect = 0.0 Identities = 937/941 (99%), Positives = 939/941 (99%)

>gb|AAF07395.1|AF106037_1 adipocyte-derived leucine aminopeptidase [Homo sapiens] Length = 941 Score = 1891 bits (4899), Expect = 0.0 Identities = 935/941 (99%), Positives = 937/941 (99%)

>gb|AAN98130.1| Sequence 353 from patent US 6478825 Length = 941 Score = 1891 bits (4898), Expect = 0.0 Identities = 935/941 (99%), Positives = 938/941 (99%)

>dbj|BAA25451.2| KIAA0525 protein [Homo sapiens] Length = 951 Score = 1887 bits (4888), Expect = 0.0 Identities = 933/939 (99%), Positives = 936/939 (99%)

>ref|NP_057526.2| type 1 tumor necrosis factor receptor shedding aminopeptidase regulator; adipocyte-derived leucine aminopeptidase; aminopeptidase PILS [Homo sapiens] gb|AAK37777.1| adipocyte-derived leucine aminopeptidase 2 isoform a [Homo sapiens] gb|AAK37778.1| adipocyte-derived leucine aminopeptidase 2 isoform b [Homo sapiens] Length = 948 Score = 1885 bits (4882), Expect = 0.0 Identities = 932/939 (99%), Positives = 935/939 (99%)

>sp|Q9NZ08|ART1_HUMAN Adipocyte-derived leucine aminopeptidase precursor (A-LAP) (ARTS-1) (Aminopeptidase PILS) (Puromycin-insensitive leucyl-specific aminopeptidase) (PILS-AP) (Type 1 tumor necrosis factor receptor shedding aminopeptidase regulator) Length = 929 Score = 1872 bits (4849), Expect = 0.0 Identities = 925/929 (99%), Positives = 927/929 (99%)

>ref|NP_109636.1| type 1 tumor necrosis factor receptor shedding aminopeptidase re; adipocyte-derived leucine aminopeptidase [Mus musculus] dbj|BAB11982.1| VEGF induced aminopeptidase [Mus musculus] dbj|BAC26904.1| unnamed protein product [Mus musculus] gb|AAH46610.1| type 1 tumor necrosis factor receptor shedding aminopeptidase regulator [Mus musculus] Length = 930 Score = 1623 bits (4202), Expect = 0.0 Identities = 791/928 (85%), Positives = 862/928 (92%), Gaps = 1/928 (0%)

>sp|Q9EQH2|ART1_MOUSE Adipocyte-derived leucine aminopeptidase precursor (A-LAP) (ARTS-1) (Aminopeptidase PILS) (Puromycin-insensitive leucyl-specific aminopeptidase) (PILS-AP) (VEGF induced aminopeptidase) gb|AAG44260.1|AF227511_1 adipocyte-derived leucine aminopeptidase [Mus musculus] Length = 930 Score = 1618 bits (4191), Expect = 0.0 Identities = 789/928 (85%), Positives = 860/928 (92%), Gaps = 1/928 (0%)

>sp|Q9JJ22|ART1_RAT Adipocyte-derived leucine aminopeptidase precursor (A-LAP) (ARTS-1) (Aminopeptidase PILS) (Puromycin-insensitive leucyl-specific aminopeptidase) (PILS-AP) gb|AAF73106.1|AF148323_1 aminopeptidase PILS [Rattus norvegicus] Length = 930 Score = 1608 bits (4164), Expect = 0.0 Identities = 784/928 (84%), Positives = 857/928 (91%), Gaps = 1/928 (0%)

>ref|NP_110463.1| leucyl-specific aminopeptidase PILS [Rattus norvegicus] gb|AAF73107.1|AF148324_1 aminopeptidase PILS [Rattus norvegicus] Length = 884 Score = 1526 bits (3950), Expect = 0.0 Identities = 744/882 (84%), Positives = 814/882 (91%), Gaps = 1/882 (0%)

>dbj|BAC30569.1| unnamed protein product [Mus musculus] Length = 694 Score = 1256 bits (3251), Expect = 0.0 Identities = 611/692 (88%), Positives = 653/692 (94%)

>gb|AAM17820.1| Sequence 1 from patent US 6362324 Length = 960 Score = 966 bits (2496), Expect = 0.0 Identities = 476/934 (50%), Positives = 646/934 (68%), Gaps = 15/934 (1%)

>ref|NP_071745.1| aminopeptidase [Homo sapiens] gb|AAG28383.|AF191545_1 aminopeptidase [Homo sapiens] Length = 960 Score = 966 bits (2496), Expect = 0.0 Identities = 476/934 (50%), Positives = 646/934 (68%), Gaps = 15/934 (1%)

>dbj|AC38159.1| unnamed protein product [Mus musculus] Length = 549 Score = 952 bits (2461), Expect = 0.0 Identities = 461/549 (83%), Positives = 507/549 (91%), Gaps = 1/549 (0%)

**Example 2: Expression profiling**

Total cellular RNA was isolated from cells by one of two standard methods: 1) guanidine isothiocyanate/Cesium chloride density gradient centrifugation [Kellogg, (1990)]; or with the Tri-Reagent protocol according to the manufacturer's specifications (Molecular Research Center, Inc., Cincinatti, Ohio). Total RNA prepared by the Tri-reagent protocol was treated with DNAse I to remove genomic DNA contamination.

For relative quantitation of the mRNA distribution of PILS, total RNA from each cell or tissue source was first reverse transcribed. 85 μg of total RNA was reverse transcribed using 1 μmole random hexamer primers, 0.5 mM each of dATP, dCTP, dGTP and dTTP (Qiagen, Hilden, Germany), 3000 U RnaseQut (Invitrogen, Groningen, Netherlands) in a final volume of 680 μl. The first strand synthesis buffer and Omniscript reverse transcriptase (2 u/μl) were from (Qiagen, Hilden, Germany). The reaction was incubated at 37°C for 90 minutes and cooled on ice. The volume was adjusted to 6800 μl with water, yielding a final concentration of 12.5 ng/μl of starting RNA.

For relative quantitation of the distribution of PILS mRNA in cells and tissues the Perkin Elmer ABI Prism RTM. 7700 Sequence Detection system or Biorad iCycler was used according to the manufacturer's specifications and protocols. PCR reactions were set up to quantitate PILS and the housekeeping genes HPRT (hypoxanthine phosphoribosyltransferase), GAPDH (glyceraldehyde-3-phosphate dehydrogenase), β-actin, and others. Forward and reverse primers and probes for PILS were designed using the Perkin Elmer ABI Primer Express™ software and were synthesized by TibMolBiol (Berlin, Germany). The PILS forward primer sequence w as: Primer1 (SEQ ID NO: 3). The PILS reverse primer sequence was Primer2 (SEQ ID NO: 4). Probe1 (SEQ ID NO: 5), labelled with FAM (carboxyfluorescein succinimidyl ester) as the reporter dye and TAMRA (carboxytetramethylrhodamine) as the quencher, is used as a probe for PILS. The following reagents were prepared in a total of 25 μl : 1x TaqMan buffer A, 5.5 mM $MgCl_2$, 200 nM of dATP, dCTP, dGTP, and dUTP, 0.025 U/μl AmpliTaq Gold™, 0.01 U/μl AmpErase and Probe1 (SEQ ID NO: 4), PILS forward and reverse primers each at 200 nM, 200 nM PILS FAM/TAMRA-labelled probe, and 5 μl of template cDNA. Thermal cycling parameters were 2 min at 50°C, followed by 10 min at 95°C, followed by 40 cycles of melting at 95°C for 15 sec and annealing/extending at 60°C for 1 min.

*Calculation of corrected CT values*

The CT (threshold cycle) value is calculated as described in the "Quantitative determination of nucleic acids" section. The CF-value (factor for threshold cycle correction) is calculated as follows :

1. PCR reactions were set up to quantitate the housekeeping genes (HKG) for each cDNA sample.

2. $CT_{HKG}$-values (threshold cycle for housekeeping gene) were calculated as described in the "Quantitative determination of nucleic acids" section.

3. $CT_{HKG}$-mean values (CT mean value of all HKG tested on one cDNAs) of all HKG for each cDNA are calculated (n = number of HKG):

$$CT_{HKG\text{-}n}\text{-mean value} = (CT_{HKG1}\text{-value} + CT_{HKG2}\text{-value} + \ldots + CT_{HKG\text{-}n}\text{-value}) / n$$

4. $CT_{pannel}$ mean value (CT mean value of all HKG in all tested cDNAs) = ($CT_{HKG1}$-mean value + $CT_{HKG2}$-mean value +...+ $CT_{HKG\text{-}y}$-mean value) / y (y = number of cDNAs)

5. $CF_{cDNA\text{-}n}$ (correction factor for cDNA n) = $CT_{pannel}$-mean value - $CT_{HKG\text{-}n}$- mean value

6. $CT_{cDNA\text{-}n}$ (CT value of the tested gene for the cDNA n) + $CF_{cDNA\text{-}n}$ (correction factor for cDNA n) = $CT_{cor\text{-}cDNA\text{-}n}$ (corrected CT value for a gene on cDNA n)

*Calculation of relative expression*

Definition : highest $CT_{cor-cDNA-n} \neq 40$ is defined as $CT_{cor-cDNA}$ [high]

Relative Expression = $2^{(CTcor\text{-}cDNA[high]\ -\ CTcor\text{-}cDNA\text{-}n)}$

*Tissues*

The expression of PILS was investigated in the tissues in table 1.

*Expression profile*

The results of the the mRNA-quantification (expression profiling) is shown in Table 1.

***Table 1**: Relative expression of PILS in various human tissues.*

| Tissue | Relative Expression |
|---|---|
| fetal heart | 15 |
| heart | 128 |
| pericardium | 47 |
| heart atrium (right) | 81 |
| heart atrium (left) | 274 |
| heart ventricle (left) | 90 |
| heart ventricle (right) | 58 |
| heart apex | 46 |
| Purkinje fibers | 64 |
| interventricular septum | 41 |
| fetal aorta | 7 |
| aorta | 46 |
| aorta sclerotic | 13 |
| artery | 5 |
| coronary artery | 3 |
| coronary artery sclerotic | 1 |
| pulmonary artery | 1 |
| carotid artery | 1 |
| mesenteric artery | 58 |
| vein | 6 |
| pulmonic valve | 10 |
| coronary artery smooth muscle primary cells | 35 |
| aortic smooth muscle cells | 19 |
| pulmonary artery smooth muscle cells | 30 |
| aortic endothel cells | 519 |
| coronary artery endothel cells | 936 |
| HUVEC cells | 18 |
| pulmonary artery endothel cells | 43 |
| iliac artery endothel cells | 90 |
| skin | 1121 |
| adrenal gland | 241 |
| thyroid | 530 |
| thyroid tumor | 67 |
| pancreas | 159 |
| pancreas liver cirrhosis | 91 |

(continued)

| Tissue | Relative |
|---|---|
| esophagus | 55 |
| esophagus tumor | 25 |
| stomach | 367 |
| stomach tumor | 82 |
| colon | 271 |
| colon tumor | 40 |
| small intestine | 564 |
| ileum | 526 |
| ileum tumor | 55 |
| ileum chronic inflammation | 43 |
| rectum | 229 |
| salivary gland | 182 |
| fetal liver | 81 |
| liver | 446 |
| liver liver cirrhosis | 744 |
| liver lupus disease | 38 |
| liver tumor | 89 |
| HEP G2 cells | 63 |
| | |
| leukocytes (peripheral blood) | 340 |
| Jurkat (T-cells) | 843 |
| bone marrow | 252 |
| erythrocytes | 36 |
| lymphnode | 13 |
| thymus | 288 |
| thrombocytes | 4 |
| bone marrow stromal cells | 48 |
| bone marrow CD71+ cells | 9 |
| bone marrow CD33+ cells | 8 |
| bone marrow CD34+ cells | 300 |
| bone marrow CD15+ cells | 12 |
| cord blood CD71+ cells | 0 |
| cord blood CD34+ cells | 322 |
| neutrophils cord blood | 137 |
| neutrophils peripheral blood | 95 |
| spleen | 209 |
| spleen liver cirrhosis | 172 |
| | |
| skeletal muscle | 106 |
| cartilage | 45 |
| bone connective tissue | 44 |
| adipose | 24 |
| | |
| fetal brain | 78 |
| brain | 187 |
| Alzheimer brain | 251 |
| cerebellum | 145 |
| Alzheimer brain cerebellum | 32 |
| cerebellum (right) | 21 |
| cerebellum (left) | 31 |

(continued)

| Tissue | Relative |
|---|---|
| cerebral cortex | 16 |
| Alzheimer cerebral cortex | 630 |
| frontal lobe | 39 |
| Alzheimer brain frontal lobe | 36 |
| occipital lobe | 380 |
| parietal lobe | 484 |
| temporal lobe | 252 |
| postcentral gyrus | 16 |
| vermis cerebelli | 8 |
| pons | 29 |
| substantia nigra | 49 |
| caudatum | 45 |
| cerebral meninges | 1 |
| cerebral peduncles | 74 |
| corpus callosum | 139 |
| nucleus accumbens | 244 |
| putamen | 127 |
| hippocampus | 307 |
| thalamus | 271 |
| posteroventral thalamus | 399 |
| dorsalmedial thalamus | 446 |
| hypothalamus | 35 |
| dorsal root ganglia | 5 |
| spinal cord | 455 |
| spinal cord (ventral horn) | 765 |
| spinal cord (dorsal horn) | 530 |
| neuroblastoma SK-N-MC cells | 17 |
| neuroblastoma SH-SY5Y cells | 234 |
| neuroblastoma IMR32 cells | 37 |
| glial tumor H4 cells | 244 |
| HEK CNS + APP | 41 |
| neural progenitor cells | 53 |
| astrocytes | 223 |
| retina | 22 |
| | |
| fetal lung | 422 |
| fetal lung fibroblast IMR-90 cells | 51 |
| fetal lung fibroblast MRC-5 cells | 39 |
| lung | 61 |
| lung right upper lobe | 207 |
| lung right mid lobe | 138 |
| lung right lower lobe | 471 |
| lung lupus disease | 31 |
| lung tumor | 14 |
| lung COPD | 91 |
| trachea | 274 |
| primary bronchia | 2226 |
| secondary bronchia | 311 |
| bronchial epithelial cells | 1342 |
| bronchial smooth muscle cells | 572 |

(continued)

| Tissue | Relative |
|---|---|
| small airway epithelial cells | 86 |
| cervix | 52 |
| testis | 96 |
| HeLa cells (cervix tumor) | 64 |
| placenta | 146 |
| uterus | 86 |
| uterus tumor | 129 |
| ovary | 755 |
| ovary tumor | 69 |
| breast | 98 |
| breast tumor | 125 |
| MDA MB 231 cells (breast tumor) | 17 |
| mammary gland | 141 |
| prostate | 307 |
| prostate BPH | 47 |
| prostate tumor | 154 |
| bladder | 320 |
| ureter | 21 |
| penis | 49 |
| corpus cavernosum | 6 |
| fetal kidney | 155 |
| kidney | 98 |
| kidney tumor | 87 |
| renal epithelial cells | 56 |
| HEK 293 cells | 33 |

**Example 3: Antisense Analysis**

Knowledge of the correct, complete cDNA sequence coding for PILS enables its use as a tool for antisense technology in the investigation of gene function. Oligonucleotides, cDNA or genomic fragments comprising the antisense strand of a polynucleotide coding for PILS are used either in vitro or in vivo to inhibit translation of the mRNA. Such technology is now well known in the art, and antisense molecules can be designed at various locations along the nucleotide sequences. By treatment of cells or whole test animals with such antisense sequences, the gene of interest is effectively turned off. Frequently, the function of the gene is ascertained by observing behavior at the intracellular, cellular, tissue or organismal level (e.g., lethality, loss of differentiated function, changes in morphology, etc.).

In addition to using sequences constructed to interrupt transcription of a particular open reading frame, modifications of gene expression is obtained by designing antisense sequences to intron regions, promoter/enhancer elements, or even to transacting regulatory genes.

**Example 4: Expression of PILS**

Expression of PILS is accomplished by subcloning the cDNAs into appropriate expression vectors and transfecting the vectors into expression hosts such as, e.g., *E. coli*. In a particular case, the vector is engineered such that it contains a promoter for β-galactosidase, upstream of the cloning site, followed by sequence containing the amino-terminal Methionine and the subsequent seven residues of β-galactosidase. Immediately following these eight residues is an engineered bacteriophage promoter useful for artificial priming and transcription and for providing a number of unique endonuclease restriction sites for cloning.

Induction of the isolated, transfected bacterial strain with Isopropyl-β-D-thiogalactopyranoside (IPTG) using standard methods produces a fusion protein corresponding to the first seven residues of β-galactosidase, about 15 residues of "linker", and the peptide encoded within the cDNA. Since cDNA clone inserts are generated by an essentially random process, there is probability of 33% that the included cDNA will lie in the correct reading frame for proper translation. If the cDNA is not in the proper reading frame, it is obtained by deletion or insertion of the appropriate number of bases using well known methods including in vitro mutagenesis, digestion with exonuclease III or mung bean nuclease, or the inclusion of an oligonucleotide linker of appropriate length.

The PILS cDNA is shuttled into other vectors known to be useful for expression of proteins in specific hosts. Oligonucleotide primers containing cloning sites as well as a segment of DNA (about 25 bases) sufficient to hybridize to stretches at both ends of the target cDNA is synthesized chemically by standard methods. These primers are then used to amplify the desired gene segment by PCR. The resulting gene segment is digested with appropriate restriction enzymes under standard conditions and isolated by gel electrophoresis. Alternately, similar gene segments are produced by digestion of the cDNA with appropriate restriction enzymes. Using appropriate primers, segments of coding sequence from more than one gene are ligated together and cloned in appropriate vectors. It is possible to optimize expression by construction of such chimeric sequences.

Suitable expression hosts for such chimeric molecules include, but are not limited to, mammalian cells such as Chinese Hamster Ovary (CHO) and human 293 cells., insect cells such as Sf9 cells, yeast cells such as *Saccharomyces cerevisiae* and bacterial cells such as *E. coli.* For each of these cell systems, a useful expression vector also includes an origin of replication to allow propagation in bacteria, and a selectable marker such as the β-lactamase antibiotic resistance gene to allow plasmid selection in bacteria. In addition, the vector may include a second selectable marker such as the neomycin phosphotransferase gene to allow selection in transfected eukaryotic host cells. Vectors for use in eukaryotic expression hosts require RNA processing elements such as 3' polyadenylation sequences if such are not part of the cDNA of interest.

Additionally, the vector contains promoters or enhancers which increase gene expression. Such promoters are host specific and include MMTV, SV40, and metallothionine promoters for CHO cells; trp, lac, tac and T7 promoters for bacterial hosts; and alpha factor, alcohol oxidase and PGH promoters for yeast. Transcription enhancers, such as the rous sarcoma virus enhancer, are used in mammalian host cells. Once homogeneous cultures of recombinant cells are obtained through standard culture methods, large quantities of recombinantly produced PILS are recovered from the conditioned medium and analyzed using chromatographic methods known in the art. For example, PILS can be cloned into the expression vector pcDNA3, as exemplified herein. This product can be used to transform, for example, HEK293 or COS by methodology standard in the art. Specifically, for example, using Lipofectamine (Gibco BRL catolog no. 1 8324-020) mediated gene transfer.

**Example 5: Isolation of Recombinant PILS**

PILS is expressed as a chimeric protein with one or more additional polypeptide domains added to facilitate protein purification. Such purification facilitating domains include, but are not limited to, metal chelating peptides such as histidine-tryptophan modules that allow purification on immobilized metals [Appa Rao, 1997] and the domain utilized in the FLAGS extension/affinity purification system (Immunex Corp., Seattle, Washington). The inclusion of a cleavable linker sequence such as Factor Xa or enterokinase (Invitrogen, Groningen, The Netherlands) between the purification domain and the PILS sequence is useful to facilitate expression of PILS.

The following example provides a method for purifying PILS.

PILS is generated using the baculovirus expression system BAC-TO-BAC (GIBCO BRL) based on *Autographa californica* nuclear polyhedrosis virus (AcNPV) infection of *Spodoptera frugiperda* insect cells (Sf9 cells).

cDNA encoding proteases cloned into either the donor plasmid pFASTBAC1 or pFASTBAC-HT which contain a mini-Tn7 transposition element. The recombinant plasmid is transformed into DH10BAC competent cells which contain the parent bacmid bMON14272 (AcNPV infectious DNA) and a helper plasmid. The mini-Tn7 element on the pFASTBAC donor can transpose to the attTn7 attachment site on the bacmid thus introducing the protease gene into the viral genome. Colonies containing recombinant bacmids are identified by disruption of the *lac*Z gene. The protease/bacmid construct can then be isolated and infected into insect cells (Sf9 cells) resulting in the production of infectious recombinant baculovirus particles and expression of either unfused recombinant enzyme (pFastbac1) or PILS-His fusion protein (pFastbacHT).

Cells are harvested and extracts prepared 24, 48 and 72 hours after transfection. Expression of PILS is confirmed by coomassie staining after sodium dodecyl sulphate-polyacrylamide gel electrophoresis (SDS-PAGE) and western blotting onto a PVDF membrane of an unstained SDS-PAGE. The protease-His fusion protein is detected due to the interaction between the Ni-NTA HRP conjugate and the His-tag which is fused to PILS.

**Example 6: Production of PILS Specific Antibodies**

Two approaches are utilized to raise antibodies to PILS, and each approach is useful for generating either polyclonal or monoclonal antibodies. In one approach, denatured protein from reverse phase HPLC separation is obtained in quantities up to 75 mg. This denatured protein is used to immunize mice or rabbits using standard protocols; about 100 μg are adequate for immunization of a mouse, while up to 1 mg might be used to immunize a rabbit. For identifying mouse hybridomas, the denatured protein is radioiodinated and used to screen potential murine B-cell hybridomas for those which produce antibody. This procedure requires only small quantities of protein, such that 20 mg is sufficient for labeling and screening of several thousand clones.

In the second approach, the amino acid sequence of an appropriate PILS domain, as deduced from translation of the cDNA, is analyzed to determine regions of high antigenicity. Oligopeptides comprising appropriate hydrophilic regions are synthesized and used in suitable immunization protocols to raise antibodies. The optimal amino acid sequences for immunization are usually at the C-terminus, the N-terminus and those intervening, hydrophilic regions of the polypeptide which are likely to be exposed to the external environment when the protein is in its natural conformation.

Typically, selected peptides, about 15 residues in length, are synthesized using an Applied Biosystems Peptide Synthesizer Model 431 A using fmoc-chemistry and coupled to keyhole limpet hemocyanin (KLH; Sigma, St. Louis, MO) by reaction with M-maleimidobenzoyl-N-hydroxysuccinimide ester, MBS. If necessary, a cysteine is introduced at the N-terminus of the peptide to permit coupling to KLH. Rabbits are immunized with the peptide-KLH complex in complete Freund's adjuvant. The resulting antisera are tested for antipeptide activity by binding the peptide to plastic, blocking with 1% bovine serum albumin, reacting with antisera, washing and reacting with labeled (radioactive or fluorescent), affinity purified, specific goat anti-rabbit IgG.

Hybridomas are prepared and screened using standard techniques. Hybridomas of interest are detected by screening with labeled PILS to identify those fusions producing the monoclonal antibody with the desired specificity. In a typical protocol, wells of plates (FAST; Becton-Dickinson, Palo Alto, CA) are coated during incubation with affinity purified, specific rabbit anti-mouse (or suitable antispecies 1 g) antibodies at 10 mg/ml. The coated wells are blocked with 1% bovine serum albumin, (BSA), washed and incubated with supernatants from hybridomas. After washing the wells are incubated with labeled PILS at 1 mg/ml. Supernatants with specific antibodies bind more labeled PILS than is detectable in the background. Then clones producing specific antibodies are expanded and subjected to two cycles of cloning at limiting dilution. Cloned hybridomas are injected into pristane-treated mice to produce ascites, and monoclonal antibody is purified from mouse ascitic fluid by affinity chromatography on Protein A. Monoclonal antibodies with affinities of at least

$10^8$ $M^{-1}$, preferably $10^9$ to $10^{10}$ $M^{-1}$ or stronger, are typically made by standard procedures.

## Example 7: Diagnostic Test Using PILS Specific Antibodies

Particular PILS antibodies are useful for investigating signal transduction and the diagnosis of infectious or hereditary conditions which are characterized by differences in the amount or distribution of PILS or downstream products of an active signaling cascade.

Diagnostic tests for PILS include methods utilizing antibody and a label to detect PILS in human body fluids, membranes, cells, tissues or extracts of such. The polypeptides and antibodies of the present invention are used with or without modification. Frequently, the polypeptides and antibodies are labeled by joining them, either covalently or noncovalently, with a substance which provides for a detectable signal. A wide variety of labels and conjugation techniques are known and have been reported extensively in both the scientific and patent literature. Suitable labels include radionuclides, enzymes, substrates, cofactors, inhibitors, fluorescent agents, chemiluminescent agents, chromogenic agents, magnetic particles and the like.

A variety of protocols for measuring soluble or membrane-bound PILS, using either polyclonal or monoclonal antibodies specific for the protein, are known in the art. Examples include enzyme-linked immunosorbent assay (ELISA), radioimmunoassay (RIA) and fluorescent activated cell sorting (FACS). A two-site monoclonal-based immunoassay utilizing monoclonal antibodies reactive to two non-interfering epitopes on PILS is preferred, but a competitive binding assay may be employed.

## Example 8: Purification of Native PILS Using Specific Antibodies

Native or recombinant PILS is purified by immunoaffinity chromatography using antibodies specific for PILS. In general, an immunoaffinity column is constructed by covalently coupling the anti-TRH antibody to an activated chromatographic resin.

Polyclonal immunoglobulins are prepared from immune sera either by precipitation with ammonium sulfate or by purification on immobilized Protein A (Pharmacia LKB Biotechnology, Piscataway N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated Sepharose (Pharmacia LKB Biotechnology). The antibody is coupled to the resin, the resin is blocked, and the derivative resin is washed according to the manufacturer's instructions.

Such immunoaffinity columns are utilized in the purification of PILS by preparing a fraction from cells containing PILS in a soluble form. This preparation is derived by solubilization of whole cells or of a subcellular fraction obtained via differential centrifugation (with or without addition of detergent) or by other methods well known in the art. Alternatively, soluble PILS containing a signal sequence is secreted in useful quantity into the medium in which the cells are grown.

A soluble PILS-containing preparation is passed over the immunoaffinity column, and the column is washed under conditions that allow the preferential absorbance of PILS (e.g., high ionic strength buffers in the presence of detergent). Then, the column is eluted under conditions that disrupt antibody/protein binding (e.g., a buffer of pH 2-3 or a high concentration of a chaotrope such as urea or thiocyanate ion), and PILS is collected.

**Example 9: Drug Screening**

This invention is particularly useful for screening therapeutic compounds by using PILS or fragments thereof in any of a variety of drug screening techniques.

The following example provides a system for drug screening measuring the protease activity.

The recombinant protease-His fusion protein can be purified from the crude lysate by metal-affinity chromatography using Ni-NTA agarose. This allows the specific retention of the recombinant material (since this is fused to the His-tag) whilst the endogenous insect proteins are washed off. The recombinant material is then eluted by competition with imidazol.

The activity of PILS molecules of the present invention can be measured using a variety of assays that measure PILS activity. For example, PILS enzyme activity can be assessed by a standard in vitro serine/metallo/... protease assay (see, for example, [U.S. 5,057,414]). Those of skill in the art are aware of a variety of substrates suitable for in vitro assays, such as SucAla-Ala-Pro-Phe-pNA, fluorescein mono-p-guanidinobenzoate hydrochloride, benzyloxycarbonyl-L-Arginyl-S-benzylester, Nalpha-Benzoyl-L-arginine ethyl ester hydrochloride, and the like. In addition, protease assay kits available from commercial sources, such as Calbiochem™ (San Diego, Calif). For general-references, see Barrett (Ed.), Methods in Enzymology, Proteolytic Enzymes: Serine and Cysteine Peptidases (Academic Press Inc. 1994), and Barrett et al., (Eds.), Handbook of Proteolytic Enzymes (Academic Press Inc. 1998).

**Example 10: Rational Drug Design**

The goal of rational drug design is to produce structural analogs of biologically active polypeptides of interest or of small molecules with which they interact, agonists, antagonists, or inhibitors. Any of these examples are used to fashion drugs which are more active or stable forms of the polypeptide or which enhance or interfere with the function of a polypeptide in vivo.

In one approach, the three-dimensional structure of a protein of interest, or of a protein-inhibitor complex, is determined by x-ray crystallography, by computer modeling or, most typically, by a combination of the two approaches. Both the shape and charges of the polypeptide must be ascertained to elucidate the structure and to determine active site(s) of the molecule. Less often, useful information regarding the structure of a polypeptide is gained by modeling based on the structure of homologous proteins. In both cases, relevant structural information is used to design efficient inhibitors. Useful examples of rational drug design include molecules which have improved activity or stability or which act as inhibitors, agonists, or antagonists of native peptides.

It is also possible to isolate a target-specific antibody, selected by functional assay, as described above, and then to solve its crystal structure. This approach, in principle, yields a pharmacore upon which subsequent drug design is based. It is possible to bypass protein crystallography altogether by generating anti-idiotypic antibodies (anti-ids) to a functional, pharmacologically active antibody. As a mirror image of a mirror image, the binding site of the anti-ids is expected to be an analog of the original receptor. The anti-id is then used to identify and isolate peptides from banks of chemically or biologically produced peptides. The isolated peptides then act as the pharmacore.

By virtue of the present invention, sufficient amount of polypeptide are made available to perform such analytical studies as X-ray crystallography. In addition, knowledge of the PILS amino acid sequence provided herein provides guidance to those employing computer modeling techniques in place of or in addition to x-ray crystallography.

**Example 11: Identification of Other Members of the Signal Transduction Complex**

Labeled PILS is useful as a reagent for the purification of molecules with which it interacts. In one embodiment of affinity purification, PILS is covalently coupled to a chromatography column. Cell-free extract derived from synovial cells or putative target cells is passed over the column, and molecules with appropriate affinity bind to PILS. PILS-complex is recovered from the column, and the PILS-binding ligand disassociated and subjected to N-terminal protein sequencing. The amino acid sequence information is then used to identify the captured molecule or to design degenerate oligonucleotide probes for cloning the relevant gene from an appropriate cDNA library.

In an alternate method, antibodies are raised against PILS, specifically monoclonal antibodies. The monoclonal antibodies are screened to identify those which inhibit the binding of labeled PILS. These monoclonal antibodies are then used therapeutically.

**Example 12: Use and Administration of Antibodies, Inhibitors, or Antagonists**

Antibodies, inhibitors, or antagonists of PILS or other treatments and compunds that are limiters of signal transduction (LSTs), provide different effects when administered therapeutically. LSTs are formulated in a nontoxic, inert, pharmaceutically acceptable aqueous carrier medium preferably at a pH of about 5 to 8, more preferably 6 to 8, although pH may vary according to the characteristics of the antibody, inhibitor, or antagonist being formulated and the condition to be treated. Characteristics of LSTs include solubility of the molecule, its half-life and antigenicity/immunogenicity. These and other characteristics aid in defining an effective carrier. Native human proteins are preferred as LSTs, but organic or synthetic molecules resulting from drug screens are equally effective in particular situations.

LSTs are delivered by known routes of administration including but not limited to topical creams and gels; transmucosal spray and aerosol; transdermal patch and bandage; injectable, intravenous and lavage formulations; and orally administered liquids and pills particularly formulated to resist stomach acid and enzymes. The particular formulation, exact dosage, and route of administration is determined by the attending physician and varies according to each specific situation.

Such determinations are made by considering multiple variables such as the condition to be treated, the LST to be administered, and the pharmacokinetic profile of a particular LST. Additional factors which are taken into account include severity of the disease state, patient's age, weight, gender and diet, time and frequency of LST administration, possible combination with other drugs, reaction sensitivities, and tolerance/response to therapy. Long acting LST formulations might be administered every 3 to 4 days, every week, or once every two weeks depending on half-life and clearance rate of the particular LST.

Normal dosage amounts vary from 0.1 to $10^5$ μg, up to a total dose of about 1 g, depending upon the route of administration. Guidance as to particular dosages and methods of delivery is provided in the literature; see U.S. P at. Nos. 4,657,760; 5,206,344; or 5,225,212. Those skilled in the art employ different formulations for different LSTs. Administration to cells such as nerve cells necessitates delivery in a manner different from that to other cells such as vascular endothelial cells.

It is contemplated that abnormal signal transduction, trauma, or diseases which trigger PILS activity are treatable with LSTs. These conditions or diseases are specifically diagnosed by the tests discussed above, and such testing should be performed in suspected cases of viral, bacterial or fungal infections, allergic responses, mechanical injury associated with trauma, hereditary diseases, lymphoma or carcinoma, or other conditions which activate the genes of lymphoid or neuronal tissues.

**Example 13: Production of Non-human Transgenic Animals**

Animal model systems which elucidate the physiological and behavioral roles of the PILS are produced by creating nonhuman transgenic animals in which the activity of the PILS is either increased or decreased, or the amino acid sequence of the expressed PILS is altered, by a variety of techniques. Examples of these techniques include, but are not limited to: 1) Insertion of normal or mutant versions of DNA encoding a PILS, by microinjection, electroporation, retroviral transfection or other means well known to those skilled in the art, into appropriately fertilized embryos in order to produce a transgenic animal or 2) homologous recombination of mutant or normal, human or animal versions of these genes with the native gene locus in transgenic animals to alter the regulation of expression or the structure of these PILS sequences. The technique of homologous recombination is well known in the art. It replaces the native gene with the inserted gene and hence is useful for producing an animal that cannot express native PILSs but does express, for example, an inserted mutant PILS, which has replaced the native PILS in the animal's genome by recombination, resulting in underexpression of the transporter. Microinjection adds genes to the genome, but does not remove them, and the technique is useful for producing an animal which expresses its own and added PILS, resulting in overexpression of the PILS.

One means available for producing a transgenic animal, with a mouse as an example, is as follows: Female mice are mated, and the resulting fertilized eggs are dissected out of their oviducts. The eggs are stored in an appropriate medium such as cesiumchloride M2 medium. DNA or cDNA encoding PILS is purified from a vector by methods well known to the one skilled in the art. Inducible promoters may be fused with the coding region of the DNA to provide an experimental means to regulate expression of the transgene. Alternatively or in addition, tissue specific regulatory elements may be fused with the coding region to permit t issue-specific expression of the transgene. The DNA, in an appropriately buffered solution, is put into a microinjection needle (which may be made from capillary tubing using a piper puller) and the egg to be injected is put in a depression slide. The needle is inserted into the pronucleus of the egg, and the DNA solution is injected. The injected egg is then transferred into the oviduct of a pseudopregnant mouse which is a mouse stimulated by the appropriate hormones in order to maintain false pregnancy, where it proceeds to the uterus, implants, and develops to term. As noted above, microinjection is not the only method for inserting DNA into the egg but is used here only for exemplary purposes.

**References**

U.S. 4,522,811
U.S. 5,057,414
U.S. 5,283,317
U.S. 5,565,332
U.S. 5,723,323
U.S. 5,747,334
U.S. 5,783,384
U.S. 5,885,814
U.S. 5,985,629
U.S. 6,362,324
U.S. 6,478,825
WO 84/03564
WO 93/03151
WO 94/13804
WO 00/73454
WO 01/22920
WO 01/64856
WO 01/90304
Akada et al., J Cell Physiol 2002 Nov;193(2):253-62
Altschul SF, Madden TL, Schaffer AA, Zhang J, Zhang Z, Miller W, Lipman DJ; Nucleic Acids Res 1997 Sep 1; 25 (17): 3389-402
Appa Rao et al., 1997, Protein Expr Purif Nov, 11(2): 201-8
Avalle et al., Ann. N Y Acad.Sci. 864:118 (1998)).
Barnes, 2000, Chest, 117:10S14S
Barrett et al., (Eds.), Handbook of Proteolytic Enzymes (Academic Press Inc. 1998).
Barrett (Ed.), Methods in Enzymology, Proteolytic Enzymes: Serine and cysteine Peptidases (Academic Press Inc. 1994)
Botstein et al., 1980, Am J Hum Genet. 32: 314-31
Colbere-Garapin et al., 1981, J. Mol. Biol. 150, 1-14
Cui, X, et al, The journal of Clinical Investigation, 2002, 110(4), p. 515-526
Cunningham and Wells, J. Mol. Biol. 234:554 (1993).
Engelhard et al., 1994, Proc. Nat. Acad. Sci. 91, 3224-3227
Friboulet et al., Appl. Biochem. Biotechnol. 47:229 (1994)
Gergen and Weiss, 1992, Am Rev Respir Dis 146:823-824
Gibson et al., 1996, Genome Research 6: 995-1001
Haseloff et al., 1988, Nature 334, 585-591
Hattori et al., J. Biochem. 125: 931-938, 1999
Hattori et al., J. Biochem. 128: 755-762, 2000
Heid et al., 1996, Genome Research 6: 986-994
Holland et al., 1991, PNAS 88: 7276-7280
Jeffreys et al., 1985, Nature 316: 76-9
Johnson et al., 1989, Endoc. Rev. 10, 317-331
Joron et al., Ann. N Y Acad. Sci. 672:216 (1992)
Karlsson, Immunol. Methods 145:229 (1991)
Kellogg et al., 1990, Anal. Biochem. 189:202-208
Lam , 1997, Anticancer Drug Res. 12(3):145-67
Livak et al., 1995 , PCR Methods and Applications 357-362
Logan, Shenk, 1984, Proc. Natl. Acad. Sci. 81, 3655-3659
Lowy et al., 1980, Cell 22, 817-23

Maddox et al., 1983, J. Exp. Med. 158, 1211-1216
Monfardini et al., Proc. Assoc. Am. Physicians 108:420 (1996)
McConnell et al., 1992 , Science 257, 1906-1912
Nicholls et al., 1993, J. Immunol. Meth. 165, 81-91
Piatak et al., 1993, BioTechniques 14:70-81
Piatak et al., 1993, Science 259:1749-1754
Porath et al., 1992, Prot. Exp. Purif. 3, 263-281
Roberge et al., 1995, Science 269, 202-204
Saric et al., Nature Immun. 3: 1169-1176, 2002
Sierevogel et al. 2003, Current Pharm. Design, Vol. 9, no. 13, pp. 1033-1040
Scott and Smith (1990) Science 249:386-390
Sjolander, Urbaniczky, 1991, Anal. Chem. 63, 2338-2345
Szabo et al., 1995, Curr. Opin. Struct. Biol. 5, 699-705
Thomas, 1980, Proc. Nat. Acad. Sci., 77:5201-5205
Tyagi, SC, 1997, Mol. Cell. Biochem. 168(1-2), 1-12
Uhlmann et al., 1987, Tetrahedron. Lett. 215, 3539-3542
Weber et al., 1990, Genomics 7: 524-30
Wigler et al., 1977, Cell 11, 223-32
Wigler et al., 1980, Proc. Natl. Acad. Sci. 77, 3567-70
York et al., Nature Immun. 3: 1177-1184, 2002

## Claims

**1.** An in vitro method of screening for therapeutic agents useful in the treatment of a disease comprised in a group of diseases consisting of heart failure, myocardial infarction, ischemic disease of the heart, arrhythmias, and atherosclerosis in coronary artery or aorta in a mammal comprising the steps of

i) determining the activity of a PILS polypeptide at a certain concentration of a test compound or in the absence of said test compound,
ii) determining the activity of said polypeptide at a different concentration of said test compound.

2. An in vitro method of screening for therapeutic agents useful in the treatment of a disease comprised in a group of diseases consisting of heart failure, myocardial infarction, ischemic diseases of the heart, arrhythmias, and atherosclerosis in coronary or aorta in a mammal comprising the steps of

   i) determining the activity of a PILS polypeptide at a certain concentration of a test compound,
   ii) determining the activity of a PILS polypeptide at the presence of a compound known to be a regulator of a PILS polypeptide.

3. A method of screening according to Claim 1 or 2 proceeded by a method of screening for therapeutic agents useful in the treatment of a disease comprised in a group of diseases consisting of heart failure, myocardial infarction, ischemic diseases of the heart, arrhythmias, and atherosclerosis in coronary artery or aorta in a mammal comprising the steps of

   i) contacting a test compound with a PILS polypeptide,
   ii) detect binding of said test compound to said PILS polypeptide.

4. The method of any of claims 1 to 3, wherein the step of contacting is in or at the surface of a cell.

5. The method of any of claims 1 to 3, wherein the cell is in vitro.

6. The method of any of claims 1 to 3, wherein the step of contacting is in a cell-free system.

7. The method of any of claims 1 to 3, wherein the polypeptide is coupled to a detectable label.

8. The method of any of claims 1 to 3, wherein the compound is coupled to a detectable label.

9. The method of any of claims 1 to 3, wherein the test compound displaces a ligand which is first bound to the polypeptide.

10. The method of any of claims 1 to 3, wherein the polypeptide is attached to a solid support.

11. The method of any of claims 1 to 3, wherein the compound is attached to a solid support.

12. An in vitro method of screening for therapeutic agents useful in the treatment of cardiovascular a disease comprised in a group of diseases consisting of heart failure, myocardial infarction, ischemic diseases of the heart, arrhythmias, and atherosclerosis in coronary artery or aorta in a mammal comprising the steps of

   i) contacting a test compound with a PILS polynucleotide,
   ii) detect binding of said test compound to said PILS polynucleotide.

13. The method of claim 12 wherein the nucleic acid molecule is RNA.

14. The method of claim 12 wherein the contacting step is in or at the surface of a cell.

15. The method of claim 12 wherein the contacting step is in a cell-free system.

16. The method of claim 12 wherein polynucleotide is coupled to a detectable label.

17. The method of claim 12 wherein the test compound is coupled to a detectable label.

18. A method of diagnosing atherosclerosis in a mammal comprising the steps of

   i) determining the amount of a PILS polynucleotide in a coronary artery or aorta sample taken from said mammal,
   ii) determining the amount of PILS polynucleotide in healthy and/or diseased mammals.

19. Use of

   i) an antisense oligonucleotide,

ii) an antibody, or
iii) a ribozyme.

which specificity regulates the activity of a PILLS polypeptide according to any claim 1 - 17 for the preparation of a medicament useful in the treatment of a disease comprised in a group of diseases consisting of heart failure, myocardial infarction, ischemic diseases, of the heart, arrhythmias, and atherosclerosis in coronary artery or aorta in a mammal.

**20.** Use of a PILS polynucleotide for the preparation of a medicament useful in the treatment of a disease comprised in a group of diseases consisting of heart failure, myocardial infarction, ischemic diseases, of the heart arrhythmias, and atherosclerosis in coronary artery or aorta in a mammal.

**21.** Use of a PILS polypeptide for the preparation of a medicament useful in the treatment of a disease comprised in a group of diseases consisting of heart failure, myocardial infarction, ischemic diseases of the heart, arrhythmias, and atherosclerosis in coronary artery or aorta in a mammal.

**Patentansprüche**

**1.** In-vitro-Verfahren zum Screening nach therapeutischen Mitteln, die in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen sind, umfassend folgende Schritte:

i) die Bestimmung der Aktivität eines PILS-Polypeptids bei einer gewissen Konzentration einer Testverbindung oder in Abwesenheit der Testverbindung,
ii) die Bestimmung der Aktivität des Polypeptids bei einer anderen Konzentration der Testverbindung.

**2.** In-vitro-Verfahren zum Screening nach therapeutischen Mitteln, die in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen sind, umfassend folgende Schritte:

i) die Bestimmung der Aktivität eines PILS-Polypeptids bei einer gewissen Konzentration einer Testverbindung,
ii) die Bestimmung der Aktivität eines PILS-Polypeptids in Gegenwart einer Verbindung, von der bekannt ist, dass sie ein PILS-Polypeptid-Regulator ist.

**3.** Verfahren zum Screening nach Anspruch 1 oder 2, dem ein Verfahren zum Screenen nach therapeutischen Mitteln vorausgeht, die in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen sind, umfassend folgende Schritte:

i) das Kontaktieren einer Testverbindung mit einem PILS-Polypeptid,
ii) das Detektieren der Bindung der Testverbindung an das PILS-Polypeptid.

**4.** Verfahren nach einem der Ansprüche 1 bis 3, worin der Schritt des Kontaktierens in einer oder an der Oberfläche einer Zelle stattfindet.

**5.** Verfahren nach einem der Ansprüche 1 bis 3, worin die Zelle in vitro vorliegt.

**6.** Verfahren nach einem der Ansprüche 1 bis 3, worin der Schritt des Kontaktierens in einem zellfreien System erfolgt.

**7.** Verfahren nach einem der Ansprüche 1 bis 3, worin das Polypeptid an eine detektierbare Markierung gebunden ist.

**8.** Verfahren nach einem der Ansprüche 1 bis 3, worin die Verbindung an eine detektierbare Markierung gebunden ist.

**9.** Verfahren nach einem der Ansprüche 1 bis 3, worin die Testverbindung einen Liganden verdrängt, der zuerst an das Polypeptid gebunden ist.

**10.** Verfahren nach einem der Ansprüche 1 bis 3, worin das Polypeptid an einen festen Träger gebunden ist.

**11.** Verfahren nach einem der Ansprüche 1 bis 3, worin die Verbindung an einen festen Träger gebunden ist.

**12.** In-vitro-Verfahren zum Screening nach therapeutischen Mitteln, die in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen sind, umfassend folgende Schritte:

i) das Kontaktieren einer Testverbindung mit einem PILS-Polynucleotid,
ii) das Detektieren der Bindung der Testverbindung an das PILS-Polynucleotid.

**13.** Verfahren nach Anspruch 12, worin das Nucleinsäuremolekül RNA ist.

**14.** Verfahren nach Anspruch 12, worin der Schritt des Kontaktierens in einer oder an der Oberfläche einer Zelle erfolgt.

**15.** Verfahren nach Anspruch 12, worin der Schritt des Kontaktierens in einem zellfreien System erfolgt.

**16.** Verfahren nach Anspruch 12, worin das Polynucleotid an eine detektierbare Markierung gebunden ist.

**17.** Verfahren nach Anspruch 12, worin die Testverbindung an eine detektierbare Markierung gebunden ist.

**18.** Verfahren zur Diagnose von Atherosklerose bei einem Säugetier, umfassend folgende Schritte:

i) die Bestimmung der Menge eines PILS-Polynucleotids in einer dem Säugetier entnommenen Koronararterien- oder Aorta-Probe,
ii) die Bestimmung der Menge an PILS-Polynucleotid bei gesunden und/oder erkrankten Säugetieren,

**19.** Verwendung

i) eines Antisense-Oligonucleotids,
ii) eines Antikörpers oder
iii) eines Ribozyms,

welche die Aktivität eines PILS-Polypeptids nach einem der Ansprüche 1 bis 17 spezifisch steuern, zur Herstellung eines Medikaments, das in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen ist.

**20.** Verwendung eines PILS-Polynucleotids zur Herstellung eines Medikaments, das in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen ist.

**21.** Verwendung eines PILS-Polypeptids zur Herstellung eines Medikaments, das in der Behandlung einer Erkrankung aus einer Gruppe von Erkrankungen, welche Herzinsuffizienz, Myokardinfarkt, ischämische Herzerkrankungen, Arrhythmien und Atherosklerose in der Koronararterie oder Aorta umfasst, bei einem Säugetier von Nutzen ist.

## Revendications

**1.** Procédé in vitro pour sélectionner des agents thérapeutiques utiles dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère, comprenant les étapes consistant à :

i) déterminer l'activité d'un polypeptide PILS à une certaine concentration d'un composé d'essai ou en l'absence dudit composé d'essai ;
ii) déterminer l'activité dudit polypeptide à une concentration différente dudit composé d'essai.

2. Procédé in vitro pour sélectionner des agents thérapeutiques utiles dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère, comprenant les étapes consistant à :

   i) déterminer l'activité d'un polypeptide PILS à une certaine concentration d'un composé d'essai ;
   ii) déterminer l'activité d'un polypeptide PILS en présence d'un composé connu en tant que régulateur d'un polypeptide PILS.

3. Procédé de sélection suivant la revendication 1 ou 2, mis en oeuvre par un procédé pour sélectionner des agents thérapeutiques utiles dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère, comprenant les étapes consistant à :

   i) mettre en contact un composé d'essai avec un polypeptide PILS ;
   ii) détecter la liaison dudit composé d'essai avec ledit polypeptide PILS.

4. Procédé suivant l'une quelconque des revendications 1 à 3, dans lequel l'étape de mise en contact est mise en oeuvre dans une, ou à la surface d'une, cellule.

5. Procédé suivant l'une quelconque des revendications 1 à 3, dans lequel la cellule est une cellule in vitro.

6. Procédé suivant l'une quelconque des revendications 1 à 3, dans lequel l'étape de mise en contact est mise en oeuvre dans un système acellulaire.

7. Procédé suivant l'une quelconque des revendications 1 à 3, dans lequel le polypeptide est couplé à un marqueur détectable.

8. Procédé suivant l'une quelconque des revendications 1 à 3, dans lequel le composé est couplé à un marqueur détectable.

9. Procédé suivant l'une quelconque des revendications 1 à 3, dans lequel le composé d'essai déplace un ligand qui est lié tout d'abord au polypeptide.

10. Procédé suivant l'une quelconque des revendications 1 à 3, dans lequel le polypeptide est fixé à un support solide.

11. Procédé suivant l'une quelconque des revendications 1 à 3, dans lequel le composé est fixé à un support solide.

12. Procédé in vitro pour sélectionner des agents thérapeutiques utiles dans le traitement d'une maladie cardio-vasculaire faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère, comprenant les étapes consistant à :

   i) mettre en contact un composé d'essai avec un polynucléotide PILS ;
   ii) détecter la liaison dudit composé d'essai audit polynucléotide PILS.

13. Procédé suivant la revendication 12, dans lequel la molécule d'acide nucléique est une molécule d'ARN.

14. Procédé suivant la revendication 12, dans lequel l'étape de mise en contact est mise en oeuvre dans une, ou à la surface d'une, cellule.

15. Procédé suivant la revendication 12, dans lequel l'étape de mise en contact est mise en oeuvre dans un système acellulaire.

16. Procédé suivant la revendication 12, dans lequel le polynucléotide est couplé à un marqueur détectable.

17. Procédé suivant la revendication 12, dans lequel le composé d'essai est couplé à un marqueur détectable.

**18.** Procédé pour diagnostiquer l'athérosclérose chez un mammifère, comprenant les étapes consistant à :

i) déterminer la quantité d'un polynucléotide PILS dans un échantillon d'artère coronaire ou d'aorte prélevé chez ledit mammifère ;
ii) déterminer la quantité de polynucléotide PILS chez des mammifère sains et/ou malades.

**19.** Utilisation

i) d'un oligonucléotide antisens,
ii) d'un anticorps ou
iii) d'un ribozyme

qui régule spécifiquement l'activité d'un polypeptide PILS suivant l'une quelconque des revendications 1 à 17, pour la préparation d'un médicament utile dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère.

**20.** Utilisation d'un polynucléotide PILS pour la préparation d'un médicament utile dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère.

**21.** Utilisation d'un polypeptide PILS pour la préparation d'un médicament utile dans le traitement d'une maladie faisant partie d'un groupe de maladies consistant en l'insuffisance cardiaque, l'infarctus du myocarde, des maladies ischémiques du coeur, les arythmies et l'athérosclérose dans l'artère coronaire ou l'aorte chez un mammifère.

**Fig. 1**

**SEQ ID NO: 1**

ATGGTGTTTCTGCCCCTCAAATGGTCCCTTGCAACCATGTCATTTCTA
CTTTCCTCACTGTTGGCTCTCTTAACTGTGTCCACTCCTTCATGGTGT
CAGAGCACTGAAGCATCTCCAAAACGTAGTGATGGGACACCATTTCCT
TGGAATAAAATACGACTTCCTGAGTACGTCATCCCAGTTCATTATGAT
CTCTTGATCCATGCAAACCTTACCACGCTGACCTTCTGGGGAACCACG
AAAGTAGAAATCACAGCCAGTCAGCCCACCAGCACCATCATCCTGCAT
AGTCACCACCTGCAGATATCTAGGGCCACCCTCAGGAAGGGAGCTGGA
GAGAGGCTATCGGAAGAACCCCTGCAGGTCCTGGAACACCCCCGTCAG
GAGCAAATTGCACTGCTGGCTCCCGAGCCCCTCCTTGTCGGGCTCCCG
TACACAGTTGTCATTCACTATGCTGGCAATCTTTCGGAGACTTTCCAC
GGATTTTACAAAAGCACCTACAGAACCAAGGAAGGGGAACTGAGGATA
CTAGCATCAACACAATTTGAACCCACTGCAGCTAGAATGGCCTTTCCC
TGCTTTGATGAACCTGCCTTCAAAGCAAGTTTCTCAATCAAAATTAGA
AGAGAGCCAAGGCACCTAGCCATCTCCAATATGCCATTGGTGAAATCT
GTGACTGTTGCTGAAGGACTCATAGAAGACCATTTTGATGTCACTGTG
AAGATGAGCACCTATCTGGTGGCCTTCATCATTTCAGATTTTGAGTCT
GTCAGCAAGATAACCAAGAGTGGAGTCAAGGTTTCTGTTTATGCTGTG
CCAGACAAGATAAATCAAGCAGATTATGCACTGGATGCTGCGGTGACT
CTTCTAGAATTTTATGAGGATTATTTCAGCATACCGTATCCCCTACCC
AAACAAGATCTTGCTGCTATTCCCGACTTTCAGTCTGGTGCTATGGAA
AACTGGGGACTGACAACATATAGAGAATCTGCTCTGTTGTTTGATGCA
GAAAAGTCTTCTGCATCAAGTAAGCTTGACATCACAATGACTGTGGCC
CATGAACTAGCCCACCAGTGGTTTGGGAACCTGGTCACTATGGAATGG
TGGAATGATCTTTGGCTAAATGAAGGATTTGCCAAATTTATGGAGTTT
GTGTCTGTCAGTGTGACCCATCCTGAACTGAAAGTTGGAGATTATTTC
TTTGGCAAATGTTTTGACGCAATGGAGGTAGATGCTTTAAATTCCTCA

CACCCTGTGTCTACACCTGTGGAAAATCCTGCTCAGATCCGGGAGATG
TTTGATGATGTTTCTTATGATAAGGGAGCTTGTATTCTGAATATGCTA
AGGGAGTATCTTAGCGCTGACGCATTTAAAAGTGGTATTGTACAGTAT
CTCCAGAAGCATAGCTATAAAAATACAAAAAACGAGGACCTGTGGGAT
AGTATGGCAAGTATTTGCCCTACAGATGGTGTAAAAGGGATGGATGGC
TTTTGCTCTAGAAGTCAACATTCATCTTCATCCTCACATTGGCATCAG
GAAAGGGTGGATGTGAAAACCATGATGAACACTTGGACACTGCAGAGG
GGTTTTCCCCTAATAACCATCACAGTGAGGGGGAGGAATGTACACATG
AAGCAAGAGCACTACATGAAGGGCTCTGACGGCGCCCCGGACACTGGG
TACCTGTGGCATGTTCCATTGACATTCATCACCAGCAAATCCGACATG
GTCCATCGATTTTTGCTAAAAACAAAAACAGATGTGCTCATCCTCCCA
GAAGAGGTGGAATGGATCAAATTTAATGTGGGCATGAATGGCTATTAC
ATTGTGCATTACGAGGATGATGGATGGGACTCTTTGACTGGCCTTTTA
AAAGGAACACACACAGCAGTCAGCAGTAATGATCGGGCAAGTCTCATT
AACAATGCATTTCAGCTCGTCAGCATTGGGAAGCTGTCCATTGAAAAG
GCCTTGGATTTATCCCTGTACTTGAAACATGAAACTGAAATTATGCCC
GTGTTTCAAGGTTTGAATGAGCTGATTCCTATGTATAAGTTAATGGAG
AAAAGAGATATGAATGAAGTGGAAACTCAATTCAAGGCCTTCCTCATC
AGGCTGCTAAGGGACCTCATTGATAAGCAGACATGGACAGACGAGGGC
TCAGTCTCAGAGCGAATGCTGCGGAGTGAACTACTACTCCTCGCCTGT
GTGCACAACTATCAGCCGTGCGTACAGAGGGCAGAAGGCTATTTCAGA
AAGTGGAAGGAATCCAATGGAAACTTGAGCCTGCCTGTCGACGTGACC
TTGGCAGTGTTTGCTGTGGGGGCCCAGAGCACAGAAGGCTGGGATTTT
CTTTATAGTAAATATCAGTTTTCTTTGTCCAGTACTGAGAAAAGCCAA
ATTGAATTTGCCCTCTGCAGAACCCAAAATAAGGAAAAGCTTCAATGG
CTACTAGATGAAAGCTTTAAGGGAGATAAAATAAAAACTCAGGAGTTT
CCACAAATTCTTACACTCATTGGCAGGAACCCAGTAGGATACCCACTG
GCCTGGCAATTTCTGAGGAAAAACTGGAACAAACTTGTACAAAAGTTT
GAACTTGGCTCATCTTCCATAGCCCACATGGTAATGGGTACAACAAAT

CAATTCTCCACAAGAACACGGCTTGAAGAGGTAAAAGGATTCTTCAGC
TCTTTGAAAGAAAATGGTTCTCAGCTCCGTTGTGTCCAACAGACAATT
GAAACCATTGAAGAAAACATCGGTTGGATGGATAAGAATTTTGATAAA
ATCAGAGTGTGGCTGCAAAGTGAAAAGCTTGAACGTATGTAA

**Fig. 2**

**SEQ ID NO: 2**

MVFLPLKWSLATMSFLLSSLLALLTVSTPSWCQSTEASPKRSDGTPFP
WNKIRLPEYVIPVHYDLLIHANLTTLTFWGTTKVEITASQPTSTIILH
SHHLQISRATLRKGAGERLSEEPLQVLEHPRQEQIALLAPEPLLVGLP
YTVVIHYAGNLSETFHGFYKSTYRTKEGELRILASTQFEPTAARMAFP
CFDEPAFKASFSIKIRREPRHLAISNMPLVKSVTVAEGLIEDHFDVTV
KMSTYLVAFIISDFESVSKITKSGVKVSVYAVPDKINQADYALDAAVT
LLEFYEDYFSIPYPLPKQDLAAIPDFQSGAMENWGLTTYRESALLFDA
EKSSASSKLDITMTVAHELAHQWFGNLVTMEWWNDLWLNEGFAKFMEF
VSVSVTHPELKVGDYFFGKCFDAMEVDALNSSHPVSTPVENPAQIREM
FDDVSYDKGACILNMLREYLSADAFKSGIVQYLQKHSYKNTKNEDLWD
SMASICPTDGVKGMDGFCSRSQHSSSSSHWHQERVDVKTMMNTWTLQR
GFPLITITVRGRNVHMKQEHYMKGSDGAPDTGYLWHVPLTFITSKSDM
VHRFLLKTKTDVLILPEEVEWIKFNVGMNGYYIVHYEDDGWDSLTGLL
KGTHTAVSSNDRASLINNAFQLVSIGKLSIEKALDLSLYLKHETEIMP
VFQGLNELIPMYKLMEKRDMNEVETQFKAFLIRLLRDLIDKQTWTDEG
SVSERMLRSELLLLACVHNYQPCVQRAEGYFRKWKESNGNLSLPVDVT
LAVFAVGAQSTEGWDFLYSKYQFSLSSTEKSQIEFALCRTQNKEKLQW
LLDESFKGDKIKTQEFPQILTLIGRNPVGYPLAWQFLRKNWNKLVQKF
ELGSSSIAHMVMGTTNQFSTRTRLEEVKGFFSSLKENGSQLRCVQQTI
ETIEENIGWMDKNFDKIRVWLQSEKLERM

**Fig. 3**

**SEQ ID NO: 3**

5'- GCTCTGACGGCGCCC -3'

**Fig. 4**

**SEQ ID NO: 4**

5'- TCGGATTTGCTGGTGATGAA -3'

**Fig. 5**

**SEQ ID NO: 5**

5'- ACACTGGGTACCTGTGGCATGTTCCA -3'

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 200164856 A
- WO 200190304 A
- WO 200122920 A
- WO 200073454 A
- US 5783384 A
- US 5747334 A
- US 5723323 A
- US 5985629 A
- US 5885814 A
- US 5565332 A
- WO 9303151 A
- WO 9413804 A
- US 5641673 A
- US 5057414 A
- WO 8403564 A
- US 5283317 A, Szabo
- US 4522811 A
- US 6478825 B
- US 6362324 B
- US 4657760 A
- US 5206344 A
- US 5225212 A
- WO 0073454 A
- WO 0122920 A
- WO 0164856 A
- WO 0190304 A

### Non-patent literature cited in the description


- **PIATAK.** *Science,* 1993
- **KAY et al.** Phage Display of Peptides and Proteins. Academic Press, 1996
- Methods in Enzymology. Proteolytic Enzymes: Serine and Cysteine Peptidases. Academic Press Inc, 1994
- Handbook of Proteolytic Enzymes. Academic Press Inc, 1998
- **ALTSCHUL SF ; MADDEN TL ; SCHAFFER AA ; ZHANG J ; ZHANG Z ; MILLER W ; LIPMAN DJ.** *Nucleic Acids Res,* 01 September 1997, vol. 25 (17), 3389-402
- *Gibco BRL catolog,* vol. 1, 8324-020
- **AKADA et al.** *J Cell Physiol,* November 2002, vol. 193 (2), 253-62
- **APPA RAO et al.** *Protein Expr Purif Nov,* 1997, vol. 11 (2), 201-8
- **AVALLE et al.** *Ann. N Y Acad.Sci.,* 1998, vol. 864, 118
- **BARNES.** *Chest,* 2000, vol. 117, 10S14S
- Methods in Enzymology. Proteolytic Enzymes: Serine and cysteine Peptidases. Academic Press Inc, 1994
- **BOTSTEIN et al.** *Am J Hum Genet.,* 1980, vol. 32, 314-31
- **COLBERE-GARAPIN et al.** *J. Mol. Biol.,* 1981, vol. 150, 1-14
- **CUI, X et al.** *The journal of Clinical Investigation,* 2002, vol. 110 (4), 515-526
- **CUNNINGHAM ; WELLS.** *J. Mol. Biol.,* 1993, vol. 234, 554
- **ENGELHARD et al.** *Proc. Nat. Acad. Sci.,* 1994, vol. 91, 3224-3227
- **FRIBOULET et al.** *Appl. Biochem. Biotechnol.,* 1994, vol. 47, 229
- **GERGEN ; WEISS.** *Am Rev Respir Dis,* 1992, vol. 146, 823-824
- **GIBSON et al.** *Genome Research,* 1996, vol. 6, 995-1001
- **HASELOFF et al.** *Nature,* 1988, vol. 334, 585-591
- **HATTORI et al.** *J. Biochem.,* 1999, vol. 125, 931-938
- **HATTORI et al.** *J. Biochem.,* 2000, vol. 128, 755-762
- **HEID et al.** *Genome Research,* 1996, vol. 6, 986-994
- **HOLLAND et al.** *PNAS,* 1991, vol. 88, 7276-7280
- **JEFFREYS et al.** *Nature,* 1985, vol. 316, 76-9
- **JOHNSON et al.** *Endoc. Rev.,* 1989, vol. 10, 317-331
- **JORON et al.** *Ann. N Y Acad. Sci.,* 1992, vol. 672, 216
- **KARLSSON.** *Immunol. Methods,* 1991, vol. 145, 229
- **KELLOGG et al.** *Anal. Biochem.,* 1990, vol. 189, 202-208
- **LAM.** *Anticancer Drug Res.,* 1997, vol. 12 (3), 145-67
- **LIVAK et al.** *PCR Methods and Applications,* 1995, 357-362

- **LOGAN ; SHENK.** *Proc. Natl. Acad. Sci.,* 1984, vol. 81, 3655-3659
- **LOWY et al.** *Cell,* 1980, vol. 22, 817-23
- **MADDOX et al.** *J. Exp. Med.,* 1983, vol. 158, 1211-1216
- **MONFARDINI et al.** *Proc. Assoc. Am. Physicians,* 1996, vol. 108, 420
- **MCCONNELL et al.** *Science,* 1992, vol. 257, 1906-1912
- **NICHOLLS et al.** *J. Immunol. Meth.,* 1993, vol. 165, 81-91
- **PIATAK et al.** *BioTechniques,* 1993, vol. 14, 70-81
- **PIATAK et al.** *Science,* 1993, vol. 259, 1749-1754
- **PORATH et al.** *Prot. Exp. Purif.,* 1992, vol. 3, 263-281
- **ROBERGE et al.** *Science,* 1995, vol. 269, 202-204
- **SARIC et al.** *Nature Immun.,* 2002, vol. 3, 1169-1176
- **SIEREVOGEL et al.** *Current Pharm. Design,* 2003, vol. 9 (13), 1033-1040
- **SCOTT ; SMITH.** *Science,* 1990, vol. 249, 386-390
- **SJOLANDER ; URBANICZKY.** *Anal. Chem.,* 1991, vol. 63, 2338-2345
- **SZABO et al.** *Curr. Opin. Struct. Biol.,* 1995, vol. 5, 699-705
- **THOMAS.** *Proc. Nat. Acad. Sci.,* 1980, vol. 77, 5201-5205
- **TYAGI, SC.** *Mol. Cell. Biochem.,* 1997, vol. 168 (1-2), 1-12
- **UHLMANN et al.** *Tetrahedron. Lett.,* 1987, vol. 215, 3539-3542
- **WEBER et al.** *Genomics,* 1990, vol. 7, 524-30
- **WIGLER et al.** *Cell,* 1977, vol. 11, 223-32
- **WIGLER et al.** *Proc. Natl. Acad. Sci.,* 1980, vol. 77, 3567-70
- **YORK et al.** *Nature Immun.,* 2002, vol. 3, 1177-1184

SEQUENCE LISTING

<110> Bayer AG, BHC

<120> Diagnostics and Therapeutics for Diseases Associated with PUROMYCIN-INSENSITIVE LEUCYL-SPECIFIC AMINOPEPTIDASE (PILS)

<130> Le A 36 744

<160> 5

<170> PatentIn version 3.1

<210> 1
<211> 2826
<212> DNA
<213> Homo sapiens

<400> 1

```
atggtgtttc tgcccctcaa atggtccctt gcaaccatgt catttctact ttcctcactg     60
ttggctctct taactgtgtc cactccttca tggtgtcaga gcactgaagc atctccaaaa    120
cgtagtgatg ggacaccatt tccttggaat aaaatacgac ttcctgagta cgtcatccca    180
gttcattatg atctcttgat ccatgcaaac cttaccacgc tgaccttctg ggaaccacg     240
aaagtagaaa tcacagccag tcagcccacc agcaccatca tcctgcatag tcaccacctg    300
cagatatcta gggccaccct caggaaggga gctggagaga ggctatcgga agaacccctg    360
caggtcctgg aacacccccg tcaggagcaa attgcactgc tggctcccga gcccctcctt    420
gtcgggctcc cgtacacagt tgtcattcac tatgctggca atctttcgga gactttccac    480
ggattttaca aaagcaccta cagaaccaag gaaggggaac tgaggatact agcatcaaca    540
caatttgaac ccactgcagc tagaatggcc tttccctgct ttgatgaacc tgccttcaaa    600
gcaagtttct caatcaaaat tagaagagag ccaaggcacc tagccatctc caatatgcca    660
ttggtgaaat ctgtgactgt tgctgaagga ctcatagaag accattttga tgtcactgtg    720
aagatgagca cctatctggt ggccttcatc atttcagatt ttgagtctgt cagcaagata    780
accaagagtg gagtcaaggt ttctgtttat gctgtgccag acaagataaa tcaagcagat    840
tatgcactgg atgctgcggt gactcttcta gaattttatg aggattattt cagcataccg    900
tatcccctac ccaaacaaga tcttgctgct attcccgact ttcagtctgg tgctatggaa    960
aactggggac tgacaacata tagagaatct gctctgttgt ttgatgcaga aaagtcttct   1020
gcatcaagta agcttgacat cacaatgact gtggcccatg aactagccca ccagtggttt   1080
gggaacctgg tcactatgga atggtggaat gatctttggc taaatgaagg atttgccaaa   1140
tttatggagt ttgtgtctgt cagtgtgacc catcctgaac tgaaagttgg agattatttc   1200
tttggcaaat gttttgacgc aatggaggta gatgctttaa attcctcaca ccctgtgtct   1260
acacctgtgg aaaatcctgc tcagatccgg gagatgtttg atgatgtttc ttatgataag   1320
ggagcttgta ttctgaatat gctaagggag tatcttagcg ctgacgcatt taaaagtggt   1380
attgtacagt atctccagaa gcatagctat aaaaatacaa aaaacgagga cctgtgggat   1440
agtatggcaa gtatttgccc tacagatggt gtaaaaggga tggatggctt ttgctctaga   1500
agtcaacatt catcttcatc ctcacattgg catcaggaaa gggtggatgt gaaaaccatg   1560
```

```
atgaacactt ggacactgca gaggggtttt cccctaataa ccatcacagt gagggggagg   1620
aatgtacaca tgaagcaaga gcactacatg aagggctctg acggcgcccc ggacactggg   1680
tacctgtggc atgttccatt gacattcatc accagcaaat ccgacatggt ccatcgattt   1740
ttgctaaaaa caaaaacaga tgtgctcatc ctcccagaag aggtggaatg gatcaaattt   1800
aatgtgggca tgaatggcta ttacattgtg cattacgagg atgatggatg ggactctttg   1860
actggccttt taaaaggaac acacacagca gtcagcagta atgatcgggc aagtctcatt   1920
aacaatgcat ttcagctcgt cagcattggg aagctgtcca ttgaaaaggc cttggattta   1980
tccctgtact tgaaacatga aactgaaatt atgcccgtgt ttcaaggttt gaatgagctg   2040
attcctatgt ataagttaat ggagaaaaga gatatgaatg aagtggaaac tcaattcaag   2100
gccttcctca tcaggctgct aagggacctc attgataagc agacatggac agacgagggc   2160
tcagtctcag agcgaatgct gcggagtgaa ctactactcc tcgcctgtgt gcacaactat   2220
cagccgtgcg tacagagggc agaaggctat ttcagaaagt ggaaggaatc caatggaaac   2280
ttgagcctgc ctgtcgacgt gaccttggca gtgtttgctg tgggggccca gagcacagaa   2340
ggctgggatt ttctttatag taaatatcag ttttctttgt ccagtactga gaaaagccaa   2400
attgaatttg ccctctgcag aacccaaaat aaggaaaagc ttcaatggct actagatgaa   2460
agctttaagg gagataaaat aaaaactcag gagtttccac aaattcttac actcattggc   2520
aggaacccag taggataccc actggcctgg caatttctga ggaaaaactg gaacaaactt   2580
gtacaaaagt ttgaacttgg ctcatcttcc atagcccaca tggtaatggg tacaacaaat   2640
caattctcca caagaacacg gcttgaagag gtaaaaggat tcttcagctc tttgaaagaa   2700
aatggttctc agctccgttg tgtccaacag acaattgaaa ccattgaaga aaacatcggt   2760
tggatggata agaattttga taaaatcaga gtgtggctgc aaagtgaaaa gcttgaacgt   2820
atgtaa                                                              2826
```

<210> 2
<211> 941
<212> PRT
<213> Homo sapiens

<400> 2

```
Met Val Phe Leu Pro Leu Lys Trp Ser Leu Ala Thr Met Ser Phe Leu
1               5                   10                  15
Leu Ser Ser Leu Leu Ala Leu Leu Thr Val Ser Thr Pro Ser Trp Cys
            20                  25                  30
Gln Ser Thr Glu Ala Ser Pro Lys Arg Ser Asp Gly Thr Pro Phe Pro
        35                  40                  45
Trp Asn Lys Ile Arg Leu Pro Glu Tyr Val Ile Pro Val His Tyr Asp
    50                  55                  60
Leu Leu Ile His Ala Asn Leu Thr Thr Leu Thr Phe Trp Gly Thr Thr
65                  70                  75                  80
Lys Val Glu Ile Thr Ala Ser Gln Pro Thr Ser Thr Ile Ile Leu His
                85                  90                  95
Ser His His Leu Gln Ile Ser Arg Ala Thr Leu Arg Lys Gly Ala Gly
            100                 105                 110
Glu Arg Leu Ser Glu Glu Pro Leu Gln Val Leu Glu His Pro Arg Gln
        115                 120                 125
```

Glu Gln Ile Ala Leu Leu Ala Pro Glu Pro Leu Leu Val Gly Leu Pro
    130                 135                 140
Tyr Thr Val Val Ile His Tyr Ala Gly Asn Leu Ser Glu Thr Phe His
145                 150                 155                 160
Gly Phe Tyr Lys Ser Thr Tyr Arg Thr Lys Glu Gly Glu Leu Arg Ile
                165                 170                 175
Leu Ala Ser Thr Gln Phe Glu Pro Thr Ala Ala Arg Met Ala Phe Pro
            180                 185                 190
Cys Phe Asp Glu Pro Ala Phe Lys Ala Ser Phe Ser Ile Lys Ile Arg
        195                 200                 205
Arg Glu Pro Arg His Leu Ala Ile Ser Asn Met Pro Leu Val Lys Ser
    210                 215                 220
Val Thr Val Ala Glu Gly Leu Ile Glu Asp His Phe Asp Val Thr Val
225                 230                 235                 240
Lys Met Ser Thr Tyr Leu Val Ala Phe Ile Ile Ser Asp Phe Glu Ser
                245                 250                 255
Val Ser Lys Ile Thr Lys Ser Gly Val Lys Val Ser Val Tyr Ala Val
            260                 265                 270
Pro Asp Lys Ile Asn Gln Ala Asp Tyr Ala Leu Asp Ala Ala Val Thr
        275                 280                 285
Leu Leu Glu Phe Tyr Glu Asp Tyr Phe Ser Ile Pro Tyr Pro Leu Pro
    290                 295                 300
Lys Gln Asp Leu Ala Ala Ile Pro Asp Phe Gln Ser Gly Ala Met Glu
305                 310                 315                 320
Asn Trp Gly Leu Thr Thr Tyr Arg Glu Ser Ala Leu Leu Phe Asp Ala
                325                 330                 335
Glu Lys Ser Ser Ala Ser Ser Lys Leu Asp Ile Thr Met Thr Val Ala
            340                 345                 350
His Glu Leu Ala His Gln Trp Phe Gly Asn Leu Val Thr Met Glu Trp
        355                 360                 365
Trp Asn Asp Leu Trp Leu Asn Glu Gly Phe Ala Lys Phe Met Glu Phe
    370                 375                 380
Val Ser Val Ser Val Thr His Pro Glu Leu Lys Val Gly Asp Tyr Phe
385                 390                 395                 400
Phe Gly Lys Cys Phe Asp Ala Met Glu Val Asp Ala Leu Asn Ser Ser
                405                 410                 415
His Pro Val Ser Thr Pro Val Glu Asn Pro Ala Gln Ile Arg Glu Met
            420                 425                 430
Phe Asp Asp Val Ser Tyr Asp Lys Gly Ala Cys Ile Leu Asn Met Leu
        435                 440                 445
Arg Glu Tyr Leu Ser Ala Asp Ala Phe Lys Ser Gly Ile Val Gln Tyr
    450                 455                 460
Leu Gln Lys His Ser Tyr Lys Asn Thr Lys Asn Glu Asp Leu Trp Asp
465                 470                 475                 480
Ser Met Ala Ser Ile Cys Pro Thr Asp Gly Val Lys Gly Met Asp Gly
                485                 490                 495

Phe Cys Ser Arg Ser Gln His Ser Ser Ser Ser Ser His Trp His Gln
500 505 510

Glu Arg Val Asp Val Lys Thr Met Met Asn Thr Trp Thr Leu Gln Arg
515 520 525

Gly Phe Pro Leu Ile Thr Ile Thr Val Arg Gly Arg Asn Val His Met
530 535 540

Lys Gln Glu His Tyr Met Lys Gly Ser Asp Gly Ala Pro Asp Thr Gly
545 550 555 560

Tyr Leu Trp His Val Pro Leu Thr Phe Ile Thr Ser Lys Ser Asp Met
565 570 575

Val His Arg Phe Leu Leu Lys Thr Lys Thr Asp Val Leu Ile Leu Pro
580 585 590

Glu Glu Val Glu Trp Ile Lys Phe Asn Val Gly Met Asn Gly Tyr Tyr
595 600 605

Ile Val His Tyr Glu Asp Asp Gly Trp Asp Ser Leu Thr Gly Leu Leu
610 615 620

Lys Gly Thr His Thr Ala Val Ser Ser Asn Asp Arg Ala Ser Leu Ile
625 630 635 640

Asn Asn Ala Phe Gln Leu Val Ser Ile Gly Lys Leu Ser Ile Glu Lys
645 650 655

Ala Leu Asp Leu Ser Leu Tyr Leu Lys His Glu Thr Glu Ile Met Pro
660 665 670

Val Phe Gln Gly Leu Asn Glu Leu Ile Pro Met Tyr Lys Leu Met Glu
675 680 685

Lys Arg Asp Met Asn Glu Val Glu Thr Gln Phe Lys Ala Phe Leu Ile
690 695 700

Arg Leu Leu Arg Asp Leu Ile Asp Lys Gln Thr Trp Thr Asp Glu Gly
705 710 715 720

Ser Val Ser Glu Arg Met Leu Arg Ser Glu Leu Leu Leu Leu Ala Cys
725 730 735

Val His Asn Tyr Gln Pro Cys Val Gln Arg Ala Glu Gly Tyr Phe Arg
740 745 750

Lys Trp Lys Glu Ser Asn Gly Asn Leu Ser Leu Pro Val Asp Val Thr
755 760 765

Leu Ala Val Phe Ala Val Gly Ala Gln Ser Thr Glu Gly Trp Asp Phe
770 775 780

Leu Tyr Ser Lys Tyr Gln Phe Ser Leu Ser Ser Thr Glu Lys Ser Gln
785 790 795 800

Ile Glu Phe Ala Leu Cys Arg Thr Gln Asn Lys Glu Lys Leu Gln Trp
805 810 815

Leu Leu Asp Glu Ser Phe Lys Gly Asp Lys Ile Lys Thr Gln Glu Phe
820 825 830

Pro Gln Ile Leu Thr Leu Ile Gly Arg Asn Pro Val Gly Tyr Pro Leu
835 840 845

Ala Trp Gln Phe Leu Arg Lys Asn Trp Asn Lys Leu Val Gln Lys Phe
850 855 860

Glu Leu Gly Ser Ser Ser Ile Ala His Met Val Met Gly Thr Thr Asn
865 870 875 880
Gln Phe Ser Thr Arg Thr Arg Leu Glu Glu Val Lys Gly Phe Phe Ser
885 890 895
Ser Leu Lys Glu Asn Gly Ser Gln Leu Arg Cys Val Gln Gln Thr Ile
900 905 910
Glu Thr Ile Glu Glu Asn Ile Gly Trp Met Asp Lys Asn Phe Asp Lys
915 920 925
Ile Arg Val Trp Leu Gln Ser Glu Lys Leu Glu Arg Met
930 935 940

<210> 3
<211> 15
<212> DNA
<213> artificial sequence

<220>
<223> forward primer

<400> 3
gctctgacgg cgccc 15

<210> 4
<211> 20
<212> DNA
<213> artificial sequence

<220>
<223> reverse primer

<400> 4
tcggatttgc tggtgatgaa 20

<210> 5
<211> 26
<212> DNA
<213> artificial sequence

<220>
<223> probe

<400> 5
acactgggta cctgtggcat gttcca 26